(19) Europäisches Patentamt
European Patent Office
Office européen des brevets

(11) **EP 3 471 745 B1**

(12) **EUROPEAN PATENT SPECIFICATION**

(45) Date of publication and mention
of the grant of the patent:
**26.03.2025 Bulletin 2025/13**

(21) Application number: **17814142.0**

(22) Date of filing: **15.06.2017**

(51) International Patent Classification (IPC):
*A61K 31/05* (2006.01)    *A61K 9/16* (2006.01)
*A61K 36/73* (2006.01)    *A61K 45/06* (2006.01)
*A61K 47/00* (2006.01)    *A61P 39/06* (2006.01)

(52) Cooperative Patent Classification (CPC):
(C-Sets available)
**A61K 31/05; A61K 9/1617; A61K 9/1652;
A61K 9/1664; A61K 36/73; A61P 39/06;**
Y02A 50/30      (Cont.)

(86) International application number:
**PCT/US2017/037797**

(87) International publication number:
**WO 2017/218853 (21.12.2017 Gazette 2017/51)**

(54) **COMPOSITIONS FOR REDUCING OXIDATIVE STRESS**

ZUSAMMENSETZUNGEN ZUR REDUZIERUNG VON OXIDATIVEM STRESS

COMPOSITIONS POUR RÉDUIRE LE STRESS OXYDATIF

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.06.2016 US 201662350702 P**

(43) Date of publication of application:
**24.04.2019 Bulletin 2019/17**

(73) Proprietor: **Ojai Energetics PBC
Ojai, CA 93023 (US)**

(72) Inventor: **KLEIDON, William
Ojai, CA 93023 (US)**

(74) Representative: **Mewburn Ellis LLP
Aurora Building
Counterslip
Bristol BS1 6BX (GB)**

(56) References cited:
**WO-A1-2013/009928    WO-A1-2013/152055
WO-A1-2016/057840    WO-A2-2009/138582
WO-A2-2015/068052    WO-A2-2016/094810**

**US-A1- 2013 004 621    US-A1- 2013 172 253
US-A1- 2015 313 868    US-A1- 2015 313 868
US-B1- 8 828 457**

• FRANCESCA COMELLI ET AL: "Beneficial
effects of a Cannabis sativa extract treatment on
diabetes-induced neuropathy and oxidative
stress", vol. 23, no. 12, 1 December 2009
(2009-12-01), pages 1678 - 1684, XP002679919,
ISSN: 0951-418X, Retrieved from the Internet
<URL:http://onlinelibrary.wiley.com/doi/10.1002/
ptr.2806/abstract> [retrieved on 20090513], DOI:
10.1002/PTR.2806
• ANONYMOUS ET AL: "How is the
Endocannabinoid... - Pennsylvania Hemp
Company | Facebook", FACEBOOK, 30 March
2016 (2016-03-30), pages 1 - 1, XP055919143,
Retrieved from the Internet <URL:https://www.
facebook.com/PennsylvaniaHempCompany/
posts/
how-is-the-endocannabinoid-system-ecs-disrup
ted-in-lyme-diseasemany-patients-bit/
501144246724686/> [retrieved on 20220509]

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

(52) Cooperative Patent Classification (CPC): (Cont.)

C-Sets
**A61K 31/05, A61K 2300/00**

**Description**

This application claims the benefit of U.S. Provisional Application No. 62/350,702, filed June 15, 2016.

**BACKGROUND**

Oxidative stress refers to an imbalance between the reactive oxygen species present within a cell and the cell's ability to neutralize the reactive oxygen species. Reactive oxygen species are characterized by a superoxide radical that can damage cellular nucleic acids, proteins, and lipids. Damage to nucleic acids such as deoxyribose nucleic acid (DNA) can include damage to bases and double stranded breaks. Damage to proteins can cause disruption to cell signaling and function. Reactive oxygen species-mediated damage to lipids can lead to disturbances in the cellular membrane.

The cell has defenses to prevent and mitigate damage by reactive oxygen species. These defenses include various enzymes and other molecules that neutralize reactive oxygen species and repair damage caused by reactive oxygen species. Examples of reactive oxygen species-neutralizing enzymes include superoxide dismutases, glutathione transferases and glutathione peroxidases. Repair enzymes destroy free-radical-damaged proteins, DNA, and oxidized fatty acids. Non-enzyme antioxidant molecules can include tocopherol, reduced glutathione, vitamin C, carotenoids, and urate. Oxidative stress can result when the aforementioned defenses are inadequate relative to an amount of reactive oxygen species in a cell.

Numerous diseases and health conditions are linked to oxidative stress. Amongst them are neurodegenerative disease, inflammation, metabolic disorders, aging, cancer, and atherosclerosis. Individuals in environments where there are high levels of radiation or carcinogens may be subject to particularly high levels of oxidative stress and hence are particularly susceptible to one or more of the aforementioned disease and health conditions. Comelli et al (Phytotherapy research. 23; 1678-1684 (2009) DOI: 10.1002/PTR.2806) describe the effects of a *cannabis sativa* extract treatment on diabetes-induced neuropathy and oxidative stress.

**SUMMARY**

There exists a considerable need for compositions and methods for reducing oxidative stress. The present disclosure addresses this need and provides other related advantages as well.

In an aspect, the invention provides a composition for use in a method for reducing oxidative stress in a subject suffering or suspected of suffering from a health condition, comprising an antioxidant and a microencapsulated cannabinoid compound and a terpene, wherein the terpene is microencapsulated with the cannabinoid compound.

In another aspect, the invention provides a food composition for use in reducing oxidative stress in a cell of a subject, comprising (a) an antioxidant and a microencapsulated cannabinoid compound and a terpene, wherein the terpene is microencapsulated with the cannabinoid compound; and (b) a food carrier.

The references to methods of treatment by therapy or surgery or in vivo diagnosis methods in this description are to be interpreted as references to compositions of the present invention for use in those methods.

In some embodiments, the terpene is quillaja *(Quillaja saponaria)*, or a derivative thereof.

In some embodiments, the health condition is selected from the group consisting of: cancer, aging, inflammation, malaria, rheumatoid arthritis, neurodegenerative diseases, diabetes, hypertension, chronic obstructive pulmonary disease, angina, arrhythmia, asthma, benign prostatic hyperplasia, carpal tunnel syndrome, bipolar disorder, cancer, cardiovascular disease, cataracts, Celiac disease, chronic fatigue syndrome, congestive heart failure, Crohn's disease, depression, dermatitis, diabetes, erectile dysfunction, fibromyalgia, gastroesophageal reflux disease, glaucoma, hypercholesterolemia, influenza, kidney stones, Lyme disease, macular degeneration, psoriasis, sleep apnea, systemic lupus erythematosus, thrombosis, and tinnitus. In some embodiments, administering the composition improves the one or more health conditions.

In some embodiments, the subject is travelling in outer space. In some embodiments, the subject is exposed to one or more type of radiation selected from the group consisting of: solar radiation, low wavelength electromagnetic radiation, galactic cosmic rays, and a combination thereof.

In some embodiments, the cannabinoid compound comprises cannabidiol (CBD). In some embodiments, the cannabinoid compound comprises less than 0.3% tetrahydrocannabinol (THC). In some embodiments, the composition further comprises an alginate.

In some embodiments, the composition is administered orally. In some embodiments, the composition is a food or beverage. In some embodiments, the composition is administered by inhalation. In some embodiments, the composition is nebulized. In some embodiments, the composition is administered transdermally.

In some embodiments, the composition further comprises one or more essential oils selected from the group consisting of: Linalool; B-Caryophyllene; B-Myrcene; D-Limonene; Humulene; a-Pinene; Ylang Ylang (Cananga odorata); Yarrow (Achillea millefolium); Violet (Viola odorata); Vetiver (Vetiveria zizanoides); Vanilla (Vanilla plantifolia); Tuberose (Polianthes tuberosa); Thyme (Thymus vulgaris L.); Tea Tree (Melaleuca alternifolia); Tangerine (Citrus reticulata); Spruce, Black (Picea mariana); Spruce (Tsuga Canadensis); Spikenard (Nardostachys jatamansi); Spearmint (Mentha spicata); Sandalwood (Santalum spicatum); Rosewood (Aniba rosaeodora); Rosemary Verbenone (Rosmarinus officinalis); Rosemary (Rosmarinus officinalis); Rose (Rosa damascena); Rose Geranium (Pelargonium roseum); Ravensara (Ravensara aromatica); Plai (Zingiber cassumunar) Pine Needle (Pinus sylvestris L.) Petitgrain (Citrus aurantium); Peppermint (Mentha piperita); Pepper, Black (Piper nigrum L.); Patchouli (Pogostemon cablin); Palo Santo (Bursera graveolens); Palmarosa (Cymbopogon martini); Osmanthus (Osmanthus fragrans); Oregano (Origanum vulgare); Orange, Sweet (Citrus sinensis); Oak Moss (Evernia prunastri); Nutmeg (Myristica fragrans) Niaouli (Melaleuca viridifloria); Neroli (aka Orange Blossom) (Citrus aurantium); Myrtle (Myrtus communis); Myrrh (Commiphora myrrha); Mimosa (Acacia decurrens); Melissa (Melissa officinalis L.); Marjoram, Sweet (Origanum majorana); Manuka (Leptospermum scoparium); Mandarin, Red (Citrus deliciosa); Mandarin (Citrus deliciosa); Lotus, White (Nelumbo nucifera); Lotus, Pink (Nelumbo nucifera); Lotus, Blue (Nelumbo nucifera); Lime (Citrus aurantifolia); Lily (Lilum aurantum); Lemongrass (Cymbopogon citratus); Lemon (Citrus limonum); Lavender (Lavandula angustifolium); Lavandin (Lavandula hybrida grosso); Kanuka (Kunzea ericoides); Juniper Berry (Juniperus cummunis); Jasmine (Jasminum officinale); Jasmine Abs (Jasminum sambac); Helichrysum (Helichrysum italicum); Grapefruit, White (Citrus x paradisi); Grapefruit, Pink (Citrus paradisi); Ginger (Zingiber officinalis); Geranium (Pelargonium graveolens); Geranium, Bourbon (Pelargonium graveolens, 'Herit); Gardenia (Gardenia jasminoides); Galbanum (Ferula galbaniflua); Frankincense (Boswellia carterii); Frangipani (Plumeria alba); Fir Needle White (Abies alba); Fir Needle Siberia (Abies siberica); Fir Needle Canada (Abies balsamea); Fennel, Sweet (Foeniculum vulgare); Eucalyptus Smithii. Eucalyptus Radiata, Eucalyptus Globulus, Eucalyptus Citriodora, Eucalyptus Blue Mallee (Eucalyptus polybractea); Elemi (Canarium luzonicum); Dill (Anethum graveolens); Cypress (Cupressus sempervirens); Cumin (Cuminum cyminum); Coriander (Coriandum sativum); Cocoa (Theobroma cacao); Clove (Eugenia caryophylatta); Clary Sage (Salvia sclarea); Cistus (aka Labdanum) ( Cistus ladaniferus L.); Cinnamon (Cinnamomum zeylanicum); Chamomile, Roman (Anthemis nobilis); Chamomile, Blue (Matricaria chamomilla); Celery Seed (Apium graveolins); Cedarwood, Western Red (Thuja plicata); Cedarwood, Blood (Juniperus virginiana); Cedarwood Atlas (Cedrus atlantica); Carrot Seed (Daucus carota); Cardamon (Elettaria cardamomum); Caraway Seed (Carum carvi); Cajeput (Melaleuca cajuputi); Cade (Juniperus oxycedrus); Birch, White (Betula alba); Birch, Sweet (Betula lenta); Bergamot (Citrus bergamia); Bay Laurel (Laurus nobilis); Basil (Ocimum basilicum); Basil, Holy (Ocimum sanctum); Basil (Ocimum basilicum); Balsam Poplar (Populus balsamifera); Balsam Peru (Myroxylon balsamum); Angelica (Angelica archangelica L.); and combinations thereof.

In some embodiments, the method can further comprise, prior to administering to the subject the composition, (i) detecting the oxidative stress in the subject, which detecting optionally comprises determining one or more levels of oxidative stress in the subject, and (ii) administering the composition to the subject based on the oxidative stress detected in (i). In some embodiments, the amount is determined based on the level determined in (i).

In some embodiments, the method can further comprise, subsequent to administering to the subject the composition, (i) determining a level of oxidative stress in the subject, and (ii) administering the composition to the subject at an additional or reduced amount or dosing regimen that is based on the level determined in (i).

In some embodiments, the method can further comprise, subsequent to administering to the subject the composition, (i) monitoring a level of oxidative stress in the subject over a period of time, and (ii) administering the composition to the subject at an additional or reduced amount or dosing regimen that is based on the level monitored in (i).

In some embodiments, the level of oxidative stress is determined or monitored upon measurement(s) of reactive oxygen species or antioxidant(s) in the subject.

In some embodiments, the food composition is packaged as a beverage. In some embodiments, the food composition is packaged as a solid food. In some embodiments, the food composition is packaged as a semi-solid food. In some embodiments, the food composition is packaged for microgravity conditions.

Useful for understanding the claimed invention, but not explicitly claimed, is a kit comprising: a food composition defined by the claims; and instructions for consumption of the food composition by the subject to effect a reduction in oxidative stress in the cell of the subject.

In some embodiments, the subject is engaged in space travel. In some embodiments, the food composition is packaged for consumption under microgravity conditions.

Additional aspects and advantages of the present disclosure will become readily apparent to those skilled in this art from the following detailed description, wherein only illustrative embodiments of the present disclosure are shown and described. The drawings and description are to be regarded as illustrative in nature, and not as restrictive.

**BRIEF DESCRIPTION OF THE DRAWINGS**

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "figure" and "FIG." herein), of which:

FIG. 1A depicts exemplary food compositions packaged for low gravity conditions;

FIG. 1B depicts exemplary beverage packaging configured for low gravity conditions;

FIG. 2A shows an exemplary microscope image of an unprocessed composition of quillaja extract, hemp oil, and water at 400x magnification;

FIG. 2B shows an exemplary microscope image of an unprocessed composition of quillaja extract, hemp oil, and water at 1000x magnification;

FIG. 3A shows an exemplary microscope image of a microfluidic processed composition of quillaja extract, hemp oil, and water at 400x magnification;

FIG. 3B shows an exemplary microscope image of a microfluidic processed composition of quillaja extract, hemp oil, and water at 400x magnification;

FIG. 3C shows an exemplary microscope image of a microfluidic processed composition of quillaja extract, hemp oil, and water at 1000x magnification;

FIG. 3D shows an exemplary microscope image of a microfluidic processed composition of quillaja extract, hemp oil, and water at 1000x magnification;

FIG. 4A shows an exemplary microscope image of a microfluidic processed composition of quillaja extract, hemp oil, water, and sodium alginate at 1000x magnification;

FIG. 4B shows an exemplary microscope image of a microfluidic processed composition of quillaja extract, hemp oil, water, and sodium alginate at 1000x magnification; and

FIG. 4C shows an exemplary microscope image of a microfluidic processed composition of quillaja extract, hemp oil, water, and sodium alginate at 1000x magnification.

## DETAILED DESCRIPTION

The references to methods of treatment by therapy or surgery or in vivo diagnosis methods in this description are to be interpreted as references to compositions of the present invention for use in those methods.

The terminology used herein is for the purpose of describing particular embodiments only and is not intended to be limiting of the invention. As used herein, the singular forms "a," "an" and "the" are intended to include the plural forms as well, unless the context clearly indicates otherwise. Furthermore, to the extent that the terms "including," "includes," "having," "has," "with," or variants thereof are used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

"Treatment," "treating," "palliating" and "ameliorating," as used herein, are used interchangeably. These terms refer to an approach for obtaining beneficial or desired results including but not limited to therapeutic benefit and/or a prophylactic benefit. Therapeutic benefit can be eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder.

In one aspect, the disclosure provides a method for administering to a subject in need thereof an effective amount of a composition as defined by the claims, thereby resulting in a reduction of oxidative stress in said subject. A subject to which a composition is administered may suffer from high oxidative stress. A subject to which a composition is administered may suffer from medium oxidative stress. A subject to which a composition is administered may suffer from low oxidative stress. A subject to which a composition is administered may suffer from endocannabinoid deficiency. For example, endocannabinoid deficiency can result from oxidative stress and/or free radical induced inflammation. In some embodiments, an encapsulated cannabinoid compound may be administered to a subject that in anticipation of oxidative stress, such as due to an activity that the subject intends to participate in or an environment that subject intends to enter. In some embodiments, an effective amount of a composition comprising an encapsulated cannabinoid compound may be administered to a subject who is exposed to high oxidative stress conditions. High oxidative stress may be the result of exogenous or endogenous stressors. Exogenous stressors may be from a subject's environment. Exogenous stressors in the environment may include various types of radiation, pollution, and chemicals. Radiation may be ionic or non-ionic. Radiation may be due to solar radiation, low wavelength electromagnetic radiation, galactic cosmic rays, and/or a combination thereof. Substances consumed by a subject, such as cigarettes, alcohol, or sugars may be sources of oxidative stress as well.

In some embodiments, such a subject may exhibit an imbalance in the production of free radicals, such as reactive oxygen species and reactive nitrogen species, and antioxidant defenses that detoxify reactive intermediates or repair free radical damage. An imbalance of free radical species and corresponding defenses may be a result of excess free radicals and/or insufficient defenses against free radicals.

Free radicals can be generated by reactive oxygen species or reactive nitrogen species. Reactive oxygen species may refer generally to oxygen radicals. Reactive nitrogen species may refer to non-radical reactive derivatives that are oxidizing agents and/ or are easily converted into radicals. Non-limiting examples of reactive oxygen species and reactive nitrogen species include radicals such as superoxide ($O_2^{\bullet-}$), hydroxyl ($OH^-$), peroxyl ($RO_2^{\bullet}$), hydroperoxyl ($HO_2^{\bullet}$), alkoxyl ($RO^{\bullet}$), peroxyl ($ROO^{\bullet}$), nitric oxide ($NO^{\bullet}$), nitrogen dioxide ($NO_2^{\bullet}$) and lipid peroxyl ($LOO^{\bullet}$) and non-radicals such as hydrogen peroxide ($H_2O_2$), hypochlorous acid ($HOCl$), ozone ($O_3$), singlet oxygen ($^1\Delta g$), peroxynitrate ($ONOO^-$), nitrous acid ($HNO_2$), dinitrogen trioxide ($N_2O_3$), and lipid peroxide ($LOOH$).

Reactive oxygen species may be generated from endogenous or environmental conditions. Regarding endogenous conditions, reactive species may be generated from various endogenous conditions. As non-limiting examples reactive oxygen species may be generated by enzymes (XO, NADPH oxidase etc.) or by auto-oxidation (e.g., adrenaline, dopamine etc.), or by leakage of electrons from the mitochondrial electron transport chain. For example, normal cellular processes such as metabolism may be a source of oxidative stress. Other sources of sources of reactive species can include: cell signaling molecules such as cytokines, radiation, osmotic shock, mechanical injury, or other environmental insults. Catalytic action of free transition metals can be a source of reactive oxygen species as well. Reactive species may result from exposure to certain chemicals (e.g., doxorubicin, cigarettes) or environmental radiation (e.g., radon, ultraviolet, ionizing).

Oxidative stress can be implicated in widespread cellular damage. Reactive oxygen species (ROS) can lead to a range of cellular responses. Low doses of reactive oxygen species can be mitogenic and increase cell proliferation. Intermediate doses of reactive oxygen species can cause temporary or permanent growth arrest. High doses of reactive oxygen species can lead to cell death by apoptosis or necrosis.

Under conditions of oxidative stress, proteins, DNA and unsaturated fatty acyl groups in membrane lipids can be chemically modified and damaged by reactive oxygen species. Accordingly, oxidative stress caused by reactive oxygen species can affect cellular response by influencing molecules involved in a variety of cell signaling pathways. Examples of pathways that may affected by reactive oxygen species under conditions of oxidative stress include MAPK pathways, ERK pathways, and c-Jun N-terminal kinases (JNK) pathway, p38 pathway, phosphoinositide 3-kinase (PI3K) pathway, phospholipase C-$\gamma$1 (PLC-$\gamma$1) signaling, Janus protein tyrosine kinase (JAK) -signal transducers and activators of transcription (STAT) (JAK-STAT) pathways, among others. These molecules modified as a result of oxidative stress can lead to changes in gene expression. Changes in gene expression may influence cell death or survival. Examples of signal transduction molecules and transcription activators affected by reactive oxygen species include: ataxia-telangectasia mutated kinase (ATM), extracellular signal-regulated kinases (ERK), heat shock transcription factor 1 (HSF1), Janus protein kinase (JAK), c-Jun N-terminal kinases (JNK), nuclear factor kB (NFkB), PI3K, phosphoinositide 3-kinase (NFkB), protein kinase C (PKC), phospholipase C- $\gamma$ 1 (PLC-$\gamma$1), and STAT.

The compositions for use provided by the invention are defined by the claims. Non-limiting examples of various health conditions include cancer, aging, inflammation, malaria, rheumatoid arthritis, neurodegenerative diseases, diabetes, hypertension, chronic obstructive pulmonary disease, angina, arrhythmia, asthma, benign prostatic hyperplasia, carpal tunnel syndrome, bipolar disorder, cancer, cardiovascular disease, cataracts, Celiac disease, chronic fatigue syndrome, congestive heart failure, Crohn's disease, depression, dermatitis, diabetes, erectile dysfunction, migraine, fibromyalgia, gastroesophageal reflux disease, glaucoma, hypercholesterolemia, influenza, kidney stones, Lyme disease, macular degeneration, psoriasis, sleep apnea, systemic lupus erythematosus, thrombosis, and tinnitus.

In some embodiments, a compound maybe administered to a subject undergoing long distance travel, including outer space travel. In some instances, long distance travel can have a travel duration of at least about 1 day, 2 days, 3 days, 4 days, 5 days, 6 days, 7 days, 1 week, 2 weeks, 3 weeks, 4 weeks, 1 month, 2 months, 3 months, 4 months, 5 months, 6 months, 7 months, 8 months, 9 months, 10 months, 11 months, 12 months, 1 year, 2 years, 3 years, 4 years, 5 years, 6 years, 7 years, 8 years, 9 years, 10 years, 20 years, 30 years, or longer. In some instances, long distance travel can have a travel duration of less than a day. Alternatively or in addition, long distance travel can have a travel distance of at least about 10, 20, 30, 40, 50, 100, 150, 200, 250, 300, 350, 400, 450, 500, 1000, 1500, 2000, 2500, 3000, 3500, 4000, 4500, 5000, 10,000, 15,000, 20,000, 25,000, 30,000, 35,000, 40,000, 45,000, 50,000, 100,000, 200,000, 300,000, 400,000, 500,000 miles (mi) or greater. In some instances, long distance travel can have a travel distance of less than 10 miles (mi). The long distance travel can be to, from, or within, partly or entirely, any environment (or atmosphere). For example, the long distance travel can involve travel to, from, or within the Earth atmosphere above or below sea level including regions of extreme low altitudes and high altitudes, underground, underwater, and/or outer space.

In some embodiments, a compound may be administered to a subject traveling in outer space. "Outer space," as disclosed herein, may refer to a region beyond the Earth's atmosphere. Outer space may refer to any region at or above the Kármán line, at an altitude of 100 km (62 mi) above sea level. Outer space may refer to any region in the thermosphere or a region at or above the mesopause. Outer space may refer to a region at or above the ozone layer. In outer space the subject may be under low gravity conditions. Low gravity conditions may refer to microgravity conditions.

The administration of an effective amount of a composition comprising an encapsulated cannabinoid compound may improve one or more health conditions in a subject disclosed herein. For example, the subject method can be used to improve health conditions including but not limited to cancer, aging, inflammation, malaria, rheumatoid arthritis, neurodegenerative diseases, diabetes, hypertension, chronic obstructive pulmonary disease, angina, arrhythmia, asthma, benign prostatic hyperplasia, carpal tunnel syndrome, bipolar disorder, cancer, cardiovascular disease, cataracts, Celiac disease, chronic fatigue syndrome, congestive heart failure, Crohn's disease, depression, dermatitis, diabetes, erectile dysfunction, migraine, fibromyalgia, gastroesophageal reflux disease, glaucoma, hypercholesterolemia, influenza, kidney stones, Lyme disease, macular degeneration, psoriasis, sleep apnea, systemic lupus erythematosus, thrombosis, and tinnitus.

In some instances, prior to administration of the composition defined by the claims, oxidative stress in a subject can be detected, assayed, and/or monitored. The composition can be administered to the subject based on the oxidative stress detected, assayed, and/or monitored. For example, the amount or dosage of the composition to be administered can be determined based on the oxidative stress detected, assayed, and/or monitored. In some instances, subsequent to administration of the composition, oxidative stress in the subject can be detected, assayed, and/or monitored. For example, a subject may be monitored for a period of time of at least about 1 minute, 2 minutes, 3 minutes, 4 minutes, 5 minutes, 10 minutes, 20 minutes. 30 minutes, 1 hour, 2 hours, 3 hours, 4 hours, 5 hours, 6 hours, 12 hours, 18 hours, 24 hours, 1 day, 2 days, 3 days, 4 days, 5 days, 6 days, 7 days, 1 week, 2 weeks, 3 weeks, 4 weeks, 1 month, 2 months, 3 months, 4 months, 5 months, 6 months, 12 months, 1 year, 2 years, 3 years, or longer. The subject may be monitored for a period of time of less than about 1 minute. Based on the oxidative stress detected, assayed and/or monitored an additional or reduced amount or dosing regimen can be administered to the subject. The presence, degree (or extent), or lack of oxidative stress may be detected by determining one or more levels of oxidative stress in the subject.

In some instances, the one or more levels of oxidative stress determined in a subject can be compared to a threshold level of oxidative stress. The threshold level of oxidative stress can be indicative of a threshold between presence and lack of oxidative stress. The threshold level of oxidative stress can be indicative of a reference level of oxidative stress, such as a level in which treatment is recommended or a level in which further monitoring is recommended. The threshold level can be unique to the subject. The threshold level can be applicable to all people, a group of people in a certain demographic (e.g., age range, sex, weight range, height range, etc.), a group of people sharing a common health condition, a group of people sharing a common treatment plan, a group of people on the same administration timeline, and/or a group of people with any shared characteristic. For example, the composition can be administered to a subject if a level of oxidative stress determined in the subject exceeds a threshold level. The administration may be halted if a level of oxidative stress determined in the subject falls below the threshold level. In another example, the amount or dosage regime of the composition administered can be increased if a level of oxidative stress determined in the subject exceeds a threshold level. The amount or dosage regime of the composition administered can be decreased if a level of oxidative stress determine din the subject falls below the threshold level.

A level of oxidative stress may be measured using a variety of techniques in the art. Some of these techniques may include direct measurement of a reduction of reactive oxygen species, measurement of resulting damage to biomolecules, detection of antioxidant levels, or a combination thereof. Reactive oxygen species can include superoxide, peroxyl radical, hydrogen peroxide, hydroxyl radical and peroxynitrite. Probes to detect oxidative stress can be used with a variety of platforms such as fluorescence microscopy, flow cytometry, or microplate analysis. Probes may be dyes that become fluorescent when oxidized or fluoresce to a different color when oxidized. One or more dyes may be used to detect a ratio of reduced to oxidized molecules. To determine a degree of oxidative stress, one or more fluorescent wavelength indicators or dyes may be compared to detect shifts in cellular oxidation. A single wavelength indicator may be used to detect reactive oxygen species.

A level of oxidative stress may be measured by detecting an amount of reactive oxygen species in a cell. Additionally, specific dyes or indicators directed to specific oxidative species may be used to assess cellular oxidative stress. Non-limiting examples of dyes include dihydroethidium, 4-amino-5-methylamino-2',7'-difluorofluorescein diacetate, and various acetoxymethyl (AM) and acetate ester derivatives of fluorescent indicators. In some instances, an amount of reactive oxygen species measured in a cell may be compared to a threshold amount of reactive oxygen species. For example, the threshold amount of reactive oxygen species can be an amount detected in a subject while the subject is not under oxidative stress.

A level of oxidative stress may be measured by detecting damage to cellular biomolecules. Damaged cellular biomolecules may include damage to protein, deoxyribose nucleic acids (DNA), ribonucleic acids (RNA), lipids or other biomolecules. Biomolecules may be detected using various assays including ELISA, immunoblots, fluorimetry, spectroscopy, and other techniques recognized by one having skill in the art.

A level of oxidative stress may be measured by assaying oxidative damage to nucleic acids. Assaying damage to nucleic acids may include assaying damage to deoxyribose nucleic acids (DNA) or ribonucleic acids (RNA). Examples of assayed biomarkers for oxidative DNA damage include 8-hydroxydeoxyguanosine (8-OHdG), apurinic or apyrimidinic (AP or abasic) sites, cyclobutane pyrimidine dimers (CPD), pyrimidine (6-4) pyrimidone photoproducts (6-4PP), double stranded breaks, or general damage to DNA molecules. Examples of assayed biomarkers for oxidative RNA damage include 8-hydroxyguanosine (8-OHG), apurinic or apyrimidinic (AP or abasic) sites. Biomarkers can be assayed using ELISA, immunofluorescence staining, probes, or other techniques recognized by one having skill in the art.

Measurement of oxidative damage to lipids may be utilized to detect a level of oxidative stress. Oxidative damage to lipids may be measured in a variety of ways. Lipid perodoxidation is a common way to measure cellular oxidative stress with respect to the cell membrane. Various techniques involving fluorimetry, ELISA, or immunoblotting may be used to determine lipid perodixation. Dyes or indicators may be configured to specifically detect lipid peroxidation. For live cells one or more indicators may be used. If more than one indicator is used, a ratiometric shift in indicator fluorescence may be indicative of an increase or decrease in oxidative stress. A single indicator may be used to detect lipid-peroxidation derived protein modifications in a fixed cell. Exemplary assays that measure lipid peroxidation may detect 4-HNE (4-hydroxynonenal), 8-iso-Prostaglandin F2a, or malondialdehyde (MDA).

A level of oxidative stress may be ascertained using assays that measure protein carbonyl content, nitrotyrosine, advanced glycation end products, advance oxidation protein end products, benzo(a)pyrene diol epoxide (BPDE) protein adducts, and others recognized by one having skill in the art.

A level of oxidative stress may be detected by assaying the activity of antioxidant molecules. Assaying antioxidant molecules assesses cellular counterbalance to various reactive oxygen species. For instance, the activity of specific antioxidant enzymes such as catalase and superoxide dismutase may be measured. A level of oxidative stress may be ascertained by detecting reduced glutathione (GSH) levels, which can be an indication of redox potential and a cell's ability to prevent oxidative stress. Some non-limiting examples of dyes that may be used to detect GSH levels include monoclorobimane (mBCl) and monobromobimane (mBBr). Ratiometric fluorescence assays may be used to assess the a balance between reactive oxygen species and antioxidants.

A level of oxidative stress may be measured in cells sampled from a subject. A level of oxidative stress may be measured a variety of biological sample types. For instance, a level of oxidative stress may be measured in cells from a biological sample type, including saliva, blood, sweat, serum, cerebrospinal fluid, plasma, urine, or any suspension of cells from a subject.

The compositions to be administered include a microencapsulated cannabinoid compound. Cannabinoids utilized in the compositions disclosed herein may include but are not limited to cannabigerol-type (CBG), cannabigerolic acid (CBGA), cannabigerolic acid monomethylether (CBGAM), cannabigerol monomethyl ether (CBGM), cannabichromene-type (CBC), cannabichromanon (CBCN), cannabichromenic acid (CBCA), cannabichromevarin-type (CBCV), cannabichromevarinic acid (CBCVA), cannabidiol-type (CBD), tetrahydrocannabinol-type (THC), iso-tetrahydrocannabinol-type (iso-THC), cannabinol-type (CBN), cannabinolic acid (CBNA), cannabinol methylether (CBNM), cannabinol-$C_4$ (CBN-$C_4$), cannabinol-$C_2$ (CBN-$C_2$), cannabiorcol (CBN-$C_1$), cannabinodiol (CBND), cannabielsoin-type (CBE), cannabielsoic acid A (CBEA-A), cannabielsoic acid B (CBEA-B), cannabicyclol-type (CBL), cannabicyclolic acid (CBLA), cannabicyclovarin (CBLV), cannabicitran-type (CBT), cannabitriol, cannabitriolvarin (CBTV), ethoxy-cannabitiolvarin (CBTVE), cannabivarin-type (CBV), cannabinodivarin (CBVD), tetrahydrocannabivarin-type (THCV), cannabidivarin-type (CBDV), cannabigerovarin-type (CBGV), cannabigerovarinic acid (CBGVA), cannabifuran (CBF), dehydrocannabifuran (DCBF), and cannabiripsol (CBR) cannabinoids.

Cannabinoids used in compositions and methods of the present disclosure can be derived from various sources, including but not limited to hemp (e.g., hemp stalk, hemp stem, hemp seed), cannabis (e.g., cannabis flower, cannabis leaf, cannabis stalk, cannabis stem, cannabis seed), Echinacea purpurea, Echinacea angustifolia, Echinacea pallida, Acmella oleracea, Helichrysum umbraculigerum, Radula marginata, kava, black truffle, Syzygium aromaticum (cloves), Rosmarinus oficinalis, basil, oregano, black pepper, lavender, true cinnamon, malabathrum, cananga odorata, copaifera spp., and hops.

Encapsulated cannabinoids can be included in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50 micrograms or more per capsule. Encapsulated cannabinoids can be included in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabinoids can be included in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabinoids can be included in a quantity of from about 1 to about 10 micrograms per capsule. Encapsulated cannabinoids can be included in a quantity of at least about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more by weight of a capsule. Encapsulated cannabinoids can be included in a quantity of at most about 0.1%, 0.2%, 0.3%, 0.4%, 0.5%, 0.6%, 0.7%,

0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated cannabinoids can be included in a quantity of about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule.

Cannabinoids can be formulated in a product, such as a food product, in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500 milligrams (mg), or more. Cannabinoids can be formulated in a product, such as a food product, in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabinoids can be formulated in a product, such as a food product, in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabinoids can be formulated in a product, such as a food product, in a quantity of from about 50 to about 150 milligrams. Cannabinoids can be formulated in a product, such as a food product, in a quantity of at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more by weight of the product. Cannabinoids can be formulated in a product, such as a food product, in a quantity of at most about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. Cannabinoids can be formulated in a product, such as a food product, in a quantity of about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product.

The cannabinoids of the encapsulated cannabinoid compounds utilized in a subject method disclosed herein can comprise cannabidiol-class compounds, including but not limited to cannabidiol (CBD), cannabidiolic acid (CBDA), cannabidiol monomethylether (CBDM), cannabidiol-$C_4$ (CBD-$C_4$), cannabidivarin (CBDV), cannabidivarinic acid (CBDVA), cannabidiorcol (CBD-$C_1$), and combinations thereof. CBD can comprise delta-1-cannabidiol, delta-2- cannabidiol, delta-3- cannabidiol, delta-3,7- cannabidiol, delta-4- cannabidiol, delta-5-cannabidiol, delta-6- cannabidiol, and combinations thereof.

Encapsulated cannabidiol compounds can be included in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50 micrograms or more per capsule. Encapsulated cannabidiol compounds can be included in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabidiol compounds can be included in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated cannabidiol compounds can be included in a quantity of from about 1 to about 10 micrograms per capsule. Encapsulated cannabidiol compounds can be included in a quantity of at least about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more by weight of a capsule. Encapsulated cannabidiol compounds can be included in a quantity of at most about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated cannabidiol compounds can be included in a quantity of about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule.

The composition comprising an encapsulated cannabinoid compound may be water soluble. The composition comprising an encapsulated cannabinoid compound may be water soluble even where atmospheric gravity is low relative to sea level. The encapsulated cannabinoid compound may be soluble at temperatures ranging from about 6°C to about 55°C. The encapsulated cannabinoid compound may be soluble at pressures ranging from about 1 atmosphere to about 5 atmospheres. The encapsulated cannabinoid compound may be soluble under ambient conditions. Ambient conditions may be about 15°C to about 30°C and about 1 atmosphere to about 3 atmospheres. For example, about 2.5 milligrams of composition may be soluble in 25 milliliters of water.

Cannabidiol compounds can be included in a product, such as a food product, in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500 milligrams (mg) or more. Cannabidiol compounds can be included in a product, such as a food product, in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabidiol compounds can be included in a product, such as a food product, in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Cannabidiol compounds can be included in a product, such as a food product, in a quantity of from about 50 to about 150 milligrams. Cannabidiol compounds can be included in a product, such as a food product, in a quantity of at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more by weight of the product. Cannabidiol compounds can be included in a product, such as a food product, in a quantity of at most about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. Cannabidiol compounds can be included in a product, such as a food product, in a quantity of about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product.

The compositions and methods of the present disclosure can comprise tetrahydrocannabinol (THC) as a type of cannabinoids. THC can comprise delta-9-THC, delta-8-THC, and combinations thereof. THC can comprise delta-6a,7-tetrahydrocannabinol, delta-7-tetrahydrocannabinol, delta-8-tetrahydrocannabinol, delta-9,11- tetrahydrocannabinol, delta-9-tetrahydrocannabinol, delta-10-tetrahydrocannabinol, delta-6a,10a- tetrahydrocannabinol, and combinations thereof. Delta-9- tetrahydrocannabinol can comprise stereoisomers including (6aR,10aR)-delta-9-tetrahydrocannabinol, (6aS,10aR)-delta-9-tetrahydrocannabinol, (6aS,10aS)-delta-9-tetrahydrocannabinol, (6aR,10aS)-delta-9-tetrahydrocannabinol, and combinations thereof.

In some embodiments, the capsules may comprise THC. THC compounds can be included in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50 micrograms or more per capsule. Encapsulated THC compounds can be included in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated THC compounds can be included in a quantity of from about 1 to about 10 micrograms per capsule. Encapsulated THC compounds can be included in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated THC compounds can be included in a quantity of at least about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more by weight of a capsule. Encapsulated THC compounds can be included in a quantity of at most about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated THC compounds can be included in a quantity of about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule.

THC compounds can be included in a product, such as a food product, in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500 milligrams (mg) or more. THC compounds can be included in a product, such as a food product, in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). THC compounds can be included in a product, such as a food product, in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). THC compounds can be included in a product, such as a food product, in a quantity of from about 50 to about 150 milligrams. THC compounds can be included in a product, such as a food product, in a quantity of at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50% or more by weight of the product. THC compounds can be included in a product, such as a food product, in a quantity of at most about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. THC compounds can be included in a product, such as a food product, in a quantity of about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product.

In some cases, a composition of the present disclosure does not contain a psychoactive amount of THC. For example, cannabinoids in compositions of the present disclosure can contain less than about 100%, 90%, 80%, 70%, 60%, 50%, 40%, 30%, 20%, 10%, 5%, 1%, 0.7%, 0.5%, 0.3%, 0.1% THC or less relative to the total quantity of cannabinoid compounds. In some cases, the ratio of a non-THC cannabinoid (e.g., cannabidiol) to THC in a composition of the present disclosure is greater than or equal to about 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 25:1, 30:1, 35:1, 40:1, 45:1, 50:1, 100:1, or greater. In some cases, compositions of the present disclosure contain less than about 0.3% THC.

The compositions comprise an antioxidant. An antioxidant may inhibit oxidation by reactive species. An antioxidant may refer to a compound that inhibits or delays the oxidation of other molecules by inhibiting the initiation or propagation of oxidizing chain reactions. An antioxidant may be a compound that neutralizes reactive oxygen species that may cause oxidative stress. An antioxidant may be compound that acts as an oxygen scavenger or a chelating agent. An antioxidant may neutralize a free radical by donating one of its own electrons. An antioxidant may be an enzymatic or non-enzymatic molecule. Enzymatic antioxidants include enzymes that metabolize oxidative toxic intermediates, such as reactive oxygen species. Enzymatic molecules include enzymes such as superoxide dismutase, glutathione peroxidase, glutathione reductase, catalases and ascorbate oxidase. Non-enzymatic molecules may include glutathione, vitamin C, uric acid, albumin, bilirubin, vitamin E ($\alpha$- tocopherol), carotenoids (e.g., $\beta$-carotene), and flavonoids.

The compositions comprise one or more terpene compounds, including but not limited to terpenoids such as monoterpenoids, sesquiterpenoids, diterpenoids, and triterpenoids. Terpenes can be acyclic, monocyclic, or polycyclic. Terpenes can include but are not limited to myrcene, limonene, linalool, trans-ocimene, cis-ocimene, alpha-pinene, beta-pinene, alpha-humulene (alpha-caryophyllene), beta-caryophyllene, delta-3-carene, trans-gamma-bisabolene, cis-gamma-bisabolene, trans-alpha-farnesene, cis-beta-farnesene, beta-fenchol, beta-phellandrene, guajol, alpha-gualene, alpha-eudesmol, beta-eudesmol, gamma-eudesmol, terpinolene, alpha-selinene, beta-selinene, alpha-terpineol, fenchone, camphene, cis-sabinene hydrate, *alpha-trans*-bergamotene, alpha-cis-bergamotene, borneol, gamma-curcumene, alpha-thujene, epi-alpha-bisabolol, ipsdienol, alpha-ylangene, beta-elemene, gamma-muurolene, alpha-cadinene, alpha-longipinene, caryophyllene oxide, and combinations thereof.

Encapsulated terpenes can be included in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50 micrograms or more per capsule. Encapsulated terpenes can be included in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated terpenes can be included in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, or 50 micrograms per capsule. Encapsulated terpene compounds can be included in a quantity of from about 1 to about 10 micrograms per capsule. Encapsulated terpenes can be included in a quantity of at least about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule. Encapsulated terpenes can be included in a quantity of at most about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, or more by weight of a capsule. Encapsulated terpenes can be included in a quantity of about 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of a capsule.

Terpene compounds can be included in a product, such as a food product, in a quantity of at least about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, 500 milligrams (mg), or more. Terpene compounds can be included in a product, such as a food product, in a quantity of at most about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Terpene compounds can be included in a product, such as a food product, in a quantity of about 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 150, 200, 250, 300, 350, 400, 450, or 500 milligrams (mg). Terpene compounds can be included in a product, such as a food product, in a quantity of from about 50 to about 150 milligrams. Terpene compounds can be included in a product, such as a food product, in a quantity of at least about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, or more by weight of the product. Terpene compounds can be included in a product, such as a food product, in a quantity of at most about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product. Terpene compounds can be included in a product, such as a food product, in a quantity of about 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% by weight of the product.

The compositions of the present disclosure can be enriched in cannabinoids compared to hemp oil. For example, a composition can comprise hemp oil and cannabinoids from plant sources such as extracts (e.g., hemp extract) and essential oils. A composition can comprise about 0%, 10%, 20%, 30%, 40%, 50%, 60%, 70%, 80%, 90%, 100%, 200%, 300%, 400%, 500%, 600%, 700%, 800%, 900%, or 1000% greater concentration of cannabinoids compared to hemp oil.

The compositions of the present disclosure can be enriched in cannabidiol compounds compared to hemp oil.

For example, a composition can comprise hemp oil and cannabidiol compounds from plant sources such as extracts (e.g., hemp extract) and essential oils. A composition can comprise about 0%, 10%, 20%, 30%, 40%, 50%, 60%, 70%, 80%, 90%, 100%, 200%, 300%, 400%, 500%, 600%, 700%, 800%, 900%, or 1000% greater concentration of cannabidiol compounds compared to hemp oil.

The compositions of the present disclosure can be enriched in THC compounds compared to hemp oil. For example, a composition can comprise hemp oil and THC compounds from plant sources such as extracts (e.g., hemp extract) and essential oils. A composition can comprise about 0%, 10%, 20%, 30%, 40%, 50%, 60%, 70%, 80%, 90%, 100%, 200%, 300%, 400%, 500%, 600%, 700%, 800%, 900%, or 1000% greater concentration of THC compounds compared to hemp oil.

The compositions of the present disclosure can be enriched in terpenes compared to hemp oil. For example, a composition can comprise hemp oil and terpenes from plant sources such as extracts (e.g., hemp extract) and essential oils. A composition can comprise about 0%, 10%, 20%, 30%, 40%, 50%, 60%, 70%, 80%, 90%, 100%, 200%, 300%, 400%, 500%, 600%, 700%, 800%, 900%, or 1000% greater concentration of terpenes compared to hemp oil.

Encapsulated cannabinoid compounds included in the compositions of the present disclosure can be derived from various sources. Compound sources can be natural, such as plant extracts or essential oils. Compounds in the compositions of the present disclosure can be derived from hemp oil, including cannabinoid compounds, THC compounds, and terpene compounds. Compounds in the compositions of the present disclosure can be derived from essential oils, including but not limited to those essential oils discussed further in this disclosure. These compounds can include cannabinoid compounds and terpene compounds. In some cases, all the compounds or ingredients in a composition are natural or naturally-derived. In some cases, all the compounds or ingredients in a composition are vegetarian. In some cases, all the compounds or ingredients in a composition are vegan.

Terpenes and/or essential oils in compositions of the present disclosure can be selected to provide benefits for particular conditions or subjects. Terpenes and/or essential oils can be employed in combination with each other, as well as in combination with cannabinoids, for example reduce cellular oxidative stress, and, in some instances, ameliorate one or more health conditions. For example, terpinolene, terpineol and linalool or lavender, valerian and jasmine essential oils can be combined with cannabinoids or cannabis extract to act as a sleep aid or treat sleep disorders. Beneficially, the combination of cannabinoids and terpenes, such as in a single composition, can have a synergistic effect on a subject's endocannabinoid system. For example, the presence of the terpenes can increase bioaccessibility of the cannabinoids. Alternatively or in addition, the presence of the cannabinoids can increase bioaccessibility of the terpenes.

Alpha-pinene can be used as an anti-inflammatory, an antiangiogenic, an anti-ulcer agent, and a bronchodilator.

Linalool can be used for reducing anxiety, reducing inflammation (e.g., lung inflammation), to improve Alzheimer's disease or symptoms thereof, as a sedative, an analgesic, an anti-microbial, an antibacterial, and an anti-epileptic.

Myrcene can be used as an antibacterial, a neuroprotective agent, an antinociceptive, an analgesic, and to alleviate neuropathic pain, peptic ulcer disease, and inflammation. Depending on concentration, myrcene can be used as a sedative (e.g., over 0.5% myrcene) or to provide energizing effects (e.g., less than 0.5% myrcene).

Limonene can be used to reduce anxiety and depression, to dissolve cholesterol-containing gallstones, to neutralize gastric acid, support normal peristalsis, relieve heartburn and gastroesophageal reflux, to improve immune function, and as a chemopreventative against cancer.

Ocimene can be used as an antifungal agent, an antitumor agent, and a cyctotoxic agent.

Terpinolene can be used for antioxidant, mood regulation, central nervous system (CNS) regulation, anti-inflammatory, anti-diarrheal, anti-filarial, anti-fungal, antimalarial, anti-amoebic, anti-bacterial, cytotoxic, and anticancer effects.

Terpineol can be used to relax a subject, to aid digestion and improve gastrointestinal disorders, and to relieve influenza, bronchitis, cough, nasal congestion, and sinusitis.

Beta-caryophyllene can be used as an anti-inflammatory agent, an anti-tumor agent, and an analgesic.

Geraniol can be used to reduce or protect against neuropathy, as an antidepressant, to suppress angiogenesis, to improve anti-cancer agent efficacy, to suppress growth of cancer cells (e.g., lung cancer), as a chemopreventive against cancer, to reduce inflammation and apoptosis (e.g., in liver cells), to reduce oxidative stress, as an antioxidant, and as an antimicrobial.

Alpha-humulene can be used as an appetite suppressant, an anti-inflammatory agent, an insect repellant, an antibacterial, an antioxidant, and an allelopathic agent.

Phellandrene can be used as an antidepressant and an antihyperalgesic.

Carene can be used as an antioxidant, an antiproliferative, an antimicrobial, and to reduce excess body fluid production, such as of tears, mucous, or sweat.

Terpinene can be used as an antioxidant, an anti-inflammatory, an antimicrobial, an antiproliferative, to reduce oxidative stress, and to manage diabetes.

Fenchol can be used as an antibacterial agent, an antimycobacterial, an antimicrobial, and an antioxidant.

Borneol can be used to alleviate hyperalgesia, as a TRPA1 inhibitor, an anti-inflammatory agent, and an anti-nociceptive agent.

Bisabolol can be used as an anti-cancer agent, such as to induce apoptosis in leukemia, an anti-tumor agent (e.g., pancreatic cancer), and an antigenotoxicity agent.

Phytol can be used to relax a subject, such as by inhibiting degradation of GABA, as an anxiolytic, to resist menadione-induced oxidative stress, and as an antimicrobial.

Camphene can be used for pain relief, as an antioxidant, to induce apoptosis in cancer cells (e.g., melanoma), an antitumor agent, and an antibacterial.

Sabinene can be used as an antioxidant, an antimicrobial, an anticancer agent (e.g., oral, liver, lung, colon, melanoma, and leukemic cancer), to aid liver function, aid digestion, relieve arthritis, and relieve skin conditions.

Camphor can be used to improve skin healing (e.g., reconstructed human epidermis), as a local anesthetic, a muscle relaxant, an antipathogenic, and an antimicrobial agent.

Isoborneol can be used as an antioxidant, a cytotoxic, a DNA-protective, to inhibit herpes simplex virus type 1, and to inhibit HIV.

Menthol can be used as an analgesic, to desensitize $\alpha3\beta4$ nicotinic acetylcholine receptors, as an antinociceptive, and as an anti-inflammatory agent.

Nerolidol can be used as an antifungal agent, an antimicrobial agent, an antioxidant, and an antimalarial agent.

Guaiol can be used as an antimicrobial agent, an antifungal agent, and an antibiotic.

Isopulegol can be used as a gastroprotective agent, an anti-inflammatory agent, to enhance permeability for transdermal administration of compounds, and to reduce the severity of seizures.

Geranyl acetate can be used as an antimicrobial agent, an antibacterial, and an antioxidant.

Cymene can be used as an anti-inflammatory agent, an anti-hyperalgesic, an antioxidant, an anti-diabetic, to aid in weight loss, to aid immune disorders, and to protect against acute lung injury.

Eucalyptol can be used as an antifungal agent, to alleviate inflammation (e.g., lung inflammation), an antioxidant, and an anticancer agent.

Pulegone can be used to enhance skin permeability, as an insecticide, and an antioxidant.

The compositions of the present disclosure can comprise one or more essential oils or essential oil compounds. Essential oils can include, but are not limited to: Linalool; B-Caryophyllene; B-Myrcene; D-Limonene; Humulene; a-Pinene; Ylang Ylang (Cananga odorata); Yarrow (Achillea millefolium); Violet (Viola odorata); Vetiver (Vetiveria zizanoides); Vanilla (Vanilla plantifolia); Tuberose (Polianthes tuberosa); Thyme (Thymus vulgaris L.); Tea Tree (Melaleuca alternifolia); Tangerine (Citrus reticulata); Spruce, Black (Picea mariana); Spruce (Tsuga Canadensis); Spikenard (Nardostachys jatamansi); Spearmint (Mentha spicata); Sandalwood (Santalum spicatum); Rosewood (Aniba rosaeodora); Rosemary Verbenone (Rosmarinus officinalis); Rosemary (Rosmarinus officinalis); Rose (Rosa damascena); Rose Geranium (Pelargonium roseum); Ravensara (Ravensara aromatica); Plai (Zingiber cassumunar) Pine Needle (Pinus sylvestris L.); Petitgrain (Citrus aurantium); Peppermint (Mentha piperita); Pepper, Black (Piper nigrum L.); Patchouli (Pogostemon cablin); Palo Santo (Bursera graveolens); Palmarosa (Cymbopogon martini); Osmanthus (Osmanthus fragrans); Oregano (Origanum vulgare); Orange, Sweet (Citrus sinensis); Oak Moss (Evernia prunastri); Nutmeg (Myristica fragrans) Niaouli (Melaleuca viridifloria); Neroli (aka Orange Blossom) (Citrus aurantium); Myrtle (Myrtus communis); Myrrh (Commiphora myrrha); Mimosa (Acacia decurrens); Melissa (Melissa officinalis L.); Marjoram, Sweet (Origanum majorana); Manuka (Leptospermum scoparium); Mandarin, Red (Citrus deliciosa); Mandarin (Citrus deliciosa); Lotus, White (Nelumbo nucifera); Lotus, Pink (Nelumbo nucifera); Lotus, Blue (Nelumbo nucifera); Lime (Citrus aurantifolia); Lily (Lilum aurantum); Lemongrass (Cymbopogon citratus); Lemon (Citrus limonum); Lavender (Lavandula angustifolium); Lavandin (Lavandula hybrida grosso); Kanuka (Kunzea ericoides); Juniper Berry (Juniperus cummunis); Jasmine (Jasminum officinale); Jasmine Abs (Jasminum sambac); Helichrysum (Helichrysum italicum); Grapefruit, White (Citrus x paradisi); Grapefruit, Pink (Citrus paradisi); Ginger (Zingiber officinalis); Geranium (Pelargonium graveolens); Geranium, Bourbon (Pelargonium graveolens, 'Herit); Gardenia (Gardenia jasminoides); Galbanum (Ferula galbaniflua); Frankincense (Boswellia carterii); Frangipani (Plumeria alba); Fir Needle White (Abies alba); Fir Needle Siberia (Abies siberica); Fir Needle Canada (Abies balsamea); Fennel, Sweet (Foeniculum vulgare); Eucalyptus Smithii. Eucalyptus Radiata, Eucalyptus Globulus, Eucalyptus Citriodora, Eucalyptus Blue Mallee (Eucalyptus polybractea); Elemi (Canarium luzonicum); Dill (Anethum graveolens); Cypress (Cupressus sempervirens); Cumin (Cuminum cyminum); Coriander (Coriandum sativum); Cocoa (Theobroma cacao); Clove (Eugenia caryophylatta); Clary Sage (Salvia sclarea); Cistus (aka Labdanum) ( Cistus ladaniferus L.); Cinnamon (Cinnamomum zeylanicum); Chamomile, Roman (Anthemis nobilis); Chamomile, Blue (Matricaria chamomilla); Celery Seed (Apium graveolins); Cedarwood, Western Red (Thuja plicata); Cedarwood, Blood (Juniperus virginiana); Cedarwood Atlas (Cedrus atlantica); Carrot Seed (Daucus carota); Cardamon (Elettaria cardamomum); Caraway Seed (Carum carvi); Cajeput (Melaleuca cajuputi); Cade (Juniperus oxycedrus); Birch, White (Betula alba); Birch, Sweet (Betula lenta); Bergamot (Citrus bergamia); Bay Laurel (Laurus nobilis); Basil (Ocimum basilicum); Basil, Holy (Ocimum sanctum); Basil (Ocimum basilicum); Balsam Poplar (Populus balsamifera); Balsam Peru (Myroxylon balsamum); Angelica (Angelica archangelica L.); and/or combinations thereof.

The compositions of the present disclosure can comprise one or more additional ingredients, including but not limited to mushrooms or mushroom derivative products (e.g., reishi mushroom, chaga mushroom, maitake mushroom, oyster mushroom, cordyceps), maca (Lepidium meyenii), he sho wu (also he show wu or shou wu chih), superfoods or superfood derivative products (e.g., blueberries, acai berries, inca berries, goji berries, camucamu, coconut, lucuma, kale, cacao (e.g., cacao powder, cacao butter), sacha inchi, chia, flax, hemp, amaranth, quinoa, moringa oleifera), and/or combinations thereof.

Compounds used in compositions of the present disclosure can be extracted by a variety of methods. For example, extraction can be performed by maceration, infusion, decoction, percolation, Soxhlet extraction, pressurized solvent extraction, counter current extraction, ultrasonication, or supercritical fluid (e.g., carbon dioxide) extraction.

In some cases, compounds used in compositions of the present disclosure are extracted via supercritical fluid (e.g., carbon dioxide) extraction. For example, cannabinoid compounds can be extracted from hemp (e.g., hemp stalk and hemp stems) using supercritical carbon dioxide extraction.

The compositions of the present disclosure can comprise pregnenolone, including derivatives thereof. Pregnenolone can help protect a subject from cannabis intoxication, for example from THC. Pregnenolone or derivatives thereof can be formulated to be water soluble. A composition of the present disclosure can comprise between about 1 and 50 milligrams (mg) of pregnenolone or derivatives thereof. For example, a unit dosage of the present disclosure can comprise between about 1 and 50 milligrams (mg) of pregnenolone. Compositions of the present disclosure (e.g., unit dosages) can comprise about 1, 2, 3, 4, 5, 6, 7, 8, 9, 10, 15, 20, 25, 30, 35, 40, 45, or 50 mg of pregnenolone. Compositions of the present disclosure (e.g., unit dosages) can comprise at least about 1, 2, 3, 4, 5, 6, 7, 8, 9, 10, 15, 20, 25, 30, 35, 40, 45, 50 mg, or more of pregnenolone. Compositions of the present disclosure (e.g., unit dosages) can comprise at most about 1, 2, 3, 4, 5, 6, 7, 8, 9, 10, 15, 20, 25, 30, 35, 40, 45, or 50 mg of pregnenolone. Compositions comprising pregnenolone can be used in combination with any other compounds, ingredients, or formulations described herein, including esters, cyclodextrin complexes, capsules (e.g., sodium alginate capsules), immediate release formulations, delayed or extended release formulations, transbuccal formulations, and sublingual formulations.

The compositions of the present disclosure comprise encapsulated cannabinoid compounds. Capsules can comprise components discussed in this disclosure, such as cannabinoid compounds. In some cases, compositions can be encapsulated without the use of liposomes. In some cases, compositions can be encapsulated without the use of micelles. In some cases, compositions can be encapsulated without the use of liposomes or micelles. Compounds of the composition can exist be encapsulated in forms including but not limited to liquid, gel, semi-solid, and solid. Encapsulated compositions disclosed herein can further be processed into forms including but not limited to solids, powders, liquids, suspensions, gels, tablets, foods, lotions, cosmetics, and other forms discussed in this disclosure.

Encapsulation can be performed with a encapsulation device, including microfluidic droplet generation or encapsulation devices. An exemplary microencapsulation device is described, for example, in U.S. Patent No. 7,482,152. Microfluidic droplets or emulsions can be generated by flow of a fluid to be encapsulated with an immiscible carrier fluid. For example, an oil fluid to be encapsulated can be flowed with an aqueous carrier fluid, or an aqueous fluid to be encapsulated can be flowed with an oil carrier fluid. Air can also be used as a fluid. Microfluidic droplet generators useful for microencapsulation include those employing co-flowing streams, cross-flowing streams (e.g., flow of streams at a T-junction), flow focusing, flow through perforated plates, and flow through nozzles. Droplet size can be controlled by parameters including device geometry, relative flow rates of the fluid streams, and operating pressure.

Examples of encapsulated compositions, including encapsulated cannabinoid compositions, are provided in WO/2016/094810.

Encapsulation can be performed at a range of operating parameters, such as different flow rates or pressures. Encapsulation can be conducted at a pressure of at least about 10 pounds per square inch (psi), 20 psi, 30 psi, 40 psi, 50 psi, 60 psi, 70 psi, 80 psi, 90 psi, 100 psi, 200 psi, 300 psi, 400 psi, 500 psi, 600 psi, 700 psi, 800 psi, 900 psi, 1000 psi, 2000 psi, 3000 psi, 4000 psi, 5000 psi, 6000 psi, 7000 psi, 8000 psi, 9000 psi, 10000 psi, 15000 psi, 20000 psi, 25000 psi, 30000 psi, 35000 psi, 40000 psi, 45000 psi, 50000 psi, or more. Encapsulation can be conducted at a pressure of at most about 10 pounds per square inch (psi), 20 psi, 30 psi, 40 psi, 50 psi, 60 psi, 70 psi, 80 psi, 90 psi, 100 psi, 200 psi, 300 psi, 400 psi, 500 psi, 600 psi, 700 psi, 800 psi, 900 psi, 1000 psi, 2000 psi, 3000 psi, 4000 psi, 5000 psi, 6000 psi, 7000 psi, 8000 psi, 9000 psi, 10000 psi, 15000 psi, 20000 psi, 25000 psi, 30000 psi, 35000 psi, 40000 psi, 45000 psi, or 50000 psi. Microencapsulation can be conducted at a pressure of about 10 pounds per square inch (psi), 20 psi, 30 psi, 40 psi, 50 psi, 60 psi, 70 psi, 80 psi, 90 psi, 100 psi, 200 psi, 300 psi, 400 psi, 500 psi, 600 psi, 700 psi, 800 psi, 900 psi, 1000 psi, 2000 psi, 3000 psi, 4000 psi, 5000 psi, 6000 psi, 7000 psi, 8000 psi, 9000 psi, 10000 psi, 15000 psi, 20000 psi, 25000 psi, 30000 psi, 35000 psi, 40000 psi, 45000 psi, 50000 psi, or more. Encapsulation can be conducted at a flow rate of at least about 1 milliliter per minute (mL/min), 2 mL/min 3 mL/min, 4 mL/min, 5 mL/min, 6 mL/min, 7 mL/min, 8 mL/min, 9 mL/min, 10 mL/min, 20 mL/min, 30 mL/min, 40 mL/min, 50 mL/min, 60 mL/min, 70 mL/min, 80 mL/min, 90 mL/min, 100 mL/min, 110 mL/min, 120 mL/min, 130 mL/min, 140 mL/min, 150 mL/min, 160 mL/min, 170 mL/min, 180 mL/min, 190 mL/min, 200 mL/min, 210 mL/min, 220 mL/min, 230 mL/min, 240 mL/min, 250 mL/min, 260 mL/min, 270 mL/min, 280 mL/min, 290 mL/min, 300 mL/min, 310 mL/min, 320 mL/min, 330 mL/min, 340 mL/min, 350 mL/min, 360 mL/min, 370 mL/min, 380 mL/min, 390 mL/min, 400 mL/min, 410 mL/min, 420 mL/min, 430 mL/min, 440 mL/min, 450 mL/min, 460 mL/min, 470 mL/min, 480 mL/min, 490 mL/min, 500 mL/min, or more. Encapsulation can be conducted at a flow rate of at most about 1 milliliter per minute (mL/min), 2 mL/min 3 mL/min, 4 mL/min, 5 mL/min, 6 mL/min, 7 mL/min, 8 mL/min, 9 mL/min, 10 mL/min, 20 mL/min, 30 mL/min, 40 mL/min, 50 mL/min, 60 mL/min, 70 mL/min, 80 mL/min, 90 mL/min, 100 mL/min, 110 mL/min, 120 mL/min, 130 mL/min, 140 mL/min, 150 mL/min, 160 mL/min, 170 mL/min, 180 mL/min, 190 mL/min, 200 mL/min, 210 mL/min, 220 mL/min, 230 mL/min, 240 mL/min, 250 mL/min, 260 mL/min, 270 mL/min, 280 mL/min, 290 mL/min, 300 mL/min, 310 mL/min, 320 mL/min, 330 mL/min, 340 mL/min, 350 mL/min, 360 mL/min, 370 mL/min, 380 mL/min, 390 mL/min, 400 mL/min, 410 mL/min, 420 mL/min, 430 mL/min, 440 mL/min, 450 mL/min, 460 mL/min, 470 mL/min, 480 mL/min, 490 mL/min, or 500 mL/min. Encapsulation can be conducted at a flow rate of about 1 milliliter per minute (mL/min), 2 mL/min 3 mL/min, 4 mL/min, 5 mL/min, 6 mL/min, 7 mL/min, 8 mL/min, 9 mL/min, 10 mL/min, 20 mL/min, 30 mL/min, 40 mL/min, 50 mL/min, 60 mL/min, 70 mL/min, 80 mL/min, 90 mL/min, 100 mL/min, 110 mL/min, 120 mL/min, 130 mL/min, 140 mL/min, 150 mL/min, 160 mL/min, 170 mL/min, 180 mL/min, 190 mL/min, 200 mL/min, 210 mL/min, 220 mL/min, 230 mL/min, 240 mL/min, 250 mL/min, 260 mL/min, 270 mL/min, 280 mL/min, 290 mL/min, 300 mL/min, 310 mL/min, 320 mL/min, 330 mL/min, 340 mL/min, 350 mL/min, 360 mL/min, 370 mL/min, 380 mL/min, 390 mL/min, 400 mL/min, 410 mL/min, 420 mL/min, 430 mL/min, 440 mL/min, 450 mL/min, 460 mL/min, 470 mL/min, 480 mL/min, 490 mL/min, 500 mL/min, or more.

Droplet generators can employ multiple parallel droplet generation operations in parallel. For example, a droplet generator (e.g., a plate, a device with channels) can employ at least about 1, 2, 3, 4, 5, 6, 7, 8, 9, 10, 15, 20, 25, 30, 35, 40, 45, 50, 55, 60, 65, 70, 75, 80, 85, 90, 95, 100, or more droplet generating features (e.g., holes, channels, nozzles). A droplet generator can employ at most about 100, 95, 90, 85, 80, 75, 70, 65, 60, 55, 50, 45, 40, 35, 30, 25, 20, 15, 10, 9, 8, 7, 6, 5, 4, 3, 2, or 1 droplet generating features. A droplet generator can employ about 1, 2, 3, 4, 5, 6, 7, 8, 9, 10, 15, 20, 25, 30, 35, 40, 45, 50, 55, 60, 65, 70, 75, 80, 85, 90, 95, 100, or more droplet generating features.

Encapsulation can be performed via an emulsification process. For example, compositions can be emulsified in a mixer, such as an agitator, impeller, centrifugal mixer, or high-shear mixer. High-shear mixers can include batch high-shear mixers and inline high-shear mixers (e.g., rotor-stator mixers). Emulsification can also be conducted without a mixer, by combining fluids thermodynamically favored to form an emulsion, optionally with the aid of one or more emulsifiers or surfactants.

Encapsulation processes can be conducted with the aid of one or more emulsifiers or surfactants. Emulsifiers and surfactants can include but are not limited to saponins (e.g., quillaja tree extract such as Q-NATURALE®, yucca extract), lecithin, soy lecithin, mustard seed hull extract, sodium stearoyl lactylate, polysorbate 20, and combinations thereof.

Encapsulated cannabinoid compounds can comprise one or more stabilizers or gelling agents, which can be used to stabilize a capsule or emulsion. Stabilizers or gelling agents can include but are not limited to alginate (also algin or alginic acid) and agar. Alginate can be used in a variety of forms, including but not limited to inorganic salts such as sodium alginate, potassium alginate, calcium alginate, and combinations thereof. Alginate can be derived from sources such as seaweed (e.g., *Macrocystis pyrifera, Ascophyllum nodosum, Laminaria* spp.) or bacteria (e.g., *Pseudomonas* spp., *Azotobacter* spp.). Cross-linking agents or solutions, such as calcium chloride, can be used to stabilize or gel capsules.

Encapsulated cannabinoid compounds can be characterized by a size (e.g., a diameter). The capsule (e.g., droplet) size can be about 0.154 micrometers. The capsule size can be less than or equal to about 0.154 micrometers. The capsule size can be greater than or equal to about 0.154 micrometers. The capsule size can be about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, 55, 60, 65, 70, 75, 80, 85, 90, 95, 100, 150, 200, 250, 300, 350, 400, 450, 500, 600, 700, 800, or 900 micrometers. The capsule size can be at most about 900, 800, 700, 600, 500, 450, 400, 350, 300, 250, 200, 150, 100, 95, 90, 85, 80, 75, 70, 65, 60, 55, 50, 45, 40, 35, 30, 25, 20, 15, 10, 9.5, 9, 8.5, 8, 7.5, 7, 6.5, 6, 5.5, 5, 4.5, 4, 3.5, 3, 2.5, 2, 1.5, 1, 0.95, 0.9, 0.85, 0.8, 0.75, 0.7, 0.65, 0.6, 0.55, 0.5, 0.45, 0.4, 0.35, 0.3, 0.25, 0.2, 0.15, 0.1, 0.09, 0.08, 0.07, 0.06, 0.05, 0.04, 0.03, 0.02, 0.01, 0.009, 0.008, 0.007, 0.006, 0.005, 0.004, 0.003, 0.002, 0.001 micrometers or less. The capsule size can be at least about 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1, 1.5, 2, 2.5, 3, 3.5, 4, 4.5, 5, 5.5, 6, 6.5, 7, 7.5, 8, 8.5, 9, 9.5, 10, 15, 20, 25, 30, 35, 40, 45, 50, 55, 60, 65, 70, 75, 80, 85, 90, 95, 100, 150, 200, 250, 300, 350, 400, 450, 500, 600, 700, 800, 900 micrometers or more. The capsule size can be from about 500 to about 900 micrometers. The capsule size can be from about 0.1 to about 0.2 micrometers. The capsule size can be from about 0.05 to about 0.25 micrometers. The capsule size can be from about 0.05 to about 0.55 micrometers. The capsule size can be from about 0.05 to about 1 micrometers. The size distribution in a population of capsules can be homogeneous or substantially homogeneous. For example, a population of capsules can be characterized by dispersity, or polydispersity index (PDI), of less than or equal to about 20, 19, 18, 17, 16, 16, 15, 14, 13, 12, 11, 10, 9, 8, 7, 6, 5, 4.9, 4.8, 4.7, 4.6, 4.5, 4.4, 4.3, 4.2, 4.1, 4.0, 3.9, 3.8, 3.7, 3.6, 3.5, 3.4, 3.3, 3.2, 3.1, 3.0, 2.9, 2.8, 2.7, 2.6, 2.5, 2.4, 2.3, 2.2, 2.1, 2.0, 1.9, 1.8, 1.7, 1.6, 1.5, 1.45, 1.40, 1.35, 1.30, 1.25, 1.20, 1.15, 1.14, 1.13, 1.12, 1.11, 1.10, 1.09, 1.08, 1.07, 1.06, 1.05, 1.04, 1.03, 1.02, 1.01, or 1.00.

An effective amount of a composition comprising an encapsulated cannabinoid compound is administered to a subject. The term "effective amount" or "therapeutically effective amount" refers to that amount of a encapsulated cannabinoid compound described herein that is sufficient to effect the intended application including but not limited to a reduction in oxidative stress in a cell and/or disease treatment in a subject. The therapeutically effective amount may vary depending upon) the subject and condition being treated, e.g., the weight and age of the subject, the severity of the disease condition, the manner of administration and the like, which can readily be determined by one of ordinary skill in the art. The term also applies to a dose that will induce a particular response in target cells, e.g., reduction in oxidative stress. The specific dose will vary depending on the particular formulation of the encapsulated cannabinoid compound, the dosing regimen to be followed, whether it is administered in combination with other compounds, timing of administration, the tissue to which it is administered, route of administration and the physical delivery system in which it is carried.

Any of the subject compositions can be provided in a unit dosage form. A unit dosage is an amount of a compound, such as a cannabinoid compound delivered alone or in combination with other components, which is to be administered to a subject at or about one time point. Other components which can be included with a unit dosage include but are not limited to cosmetics, food carriers, food bars, baked goods, dairy products, oils, beverages, solid dosages (e.g., tablets), or liquid dosages. A unit dosage of a cannabinoid compound can be about 10, 20, 30, 40, 50, 60, 70, 80, 90, 100, 125, 150, 175, 200, 250, 300, 350, 400, 450, 500, 600, 700, 800, 900, 1000, 1100, 1200, 1300, 1400, 1500, 1600, 1700, 1800, 1900, 2000 or more milligrams (mg). A unit dosage of a cannabinoid compound can be at least about 10, 20, 30, 40, 50, 60, 70, 80, 90, 100, 125, 150, 175, 200, 250, 300, 350, 400, 450, 500, 600, 700, 800, 900, 1000, 1100, 1200, 1300, 1400, 1500, 1600, 1700, 1800, 1900, 2000 or more milligrams (mg). A unit dosage of a cannabinoid compound can be at most about 2000, 1900, 1800, 1700, 1600, 1500, 1400, 1300, 1200, 1100, 1000, 900, 800, 700, 600, 500, 450, 400, 350, 300, 250, 200, 175, 150, 125, 100, 90, 80, 70, 60, 50, 40, 30, 20, 10, or less milligrams (mg). A unit dosage can be an hourly dosage. A unit dosage can be a daily dosage. A unit dosage can provide about 1/24, 1/12, 1/8, 1/6, 1/4, 1/3, 1/2, or all of a daily dosage of one or more cannabinoids for a subject. A unit dosage can take the form of a tablet, gel, liquid, food product, food bar, container of liquid of defined volume, or other forms described herein, packaged for one-time consumption or administration.

The amount of the composition of the subject method administered will be dependent on the subject being treated, the severity of disorder or condition, the rate of administration, the disposition of the composition and encapsulated cannabinoid compound. An effective dosage is in the range of about 0.1 mg to about 2000 mg per kg body weight per day. For example, for a 70 kg human, this may amount to about 7 mg/day to about 1.75 g/day. In some instances, dosage levels below the lower limit of the aforesaid range may be more than adequate, while in other case still larger doses may be employed without causing any harmful side effects, e.g.,by dividing such larger doses in several small doses for administration throughout the day. Dosages may be administered over time periods of hours, days, weeks, or months.

The composition comprising an encapsulated cannabinoid compound can be administered to a subject in any of several ways to effect a reduction in oxidative stress. The compositions can be administered, for example, orally or transdermally. Other delivery routes include but are not limited to intranasal, sublingual, transmucosal, or intradermal. An effective amount of the compositions may be in in dosage unit formulations containing conventional nontoxic physiologically acceptable carriers, adjuvants, and vehicles as desired.

Oral administration may be accomplished using an oral dosage form. Oral dosage forms can be solid (e.g., a tablet or bulk powder) or liquid (e.g., a suspension or slurry). Tablets can include tablets, caplets, capsules, including soft gelatin capsules, and lozenges. Tablets can further comprise suitable binders, lubricants, diluents, disintegrating agents, colorants, flavoring agents, flow-inducing agents, and melting agents.

Compositions comprising an encapsulated cannabinoid compound formulated for oral administration can be incorporated into a food composition. A food composition can be a beverage, a solid food, or a semi-solid food. Food compositions can comprise an encapsulated cannabinoid compound and a food carrier. A food carrier can be practically any food product. Examples of food carriers include, but are not limited to food bars (granola bars, protein bars, candy bars, etc.), cereal products (oatmeal, breakfast cereals, granola, etc.), bakery products (bread, donuts, crackers, bagels, pastries, cakes, etc.), beverages (milk-based beverage, sports drinks, fruit juices, alcoholic beverages, bottled waters), pastas, grains (rice, corn, oats, rye, wheat, flour, etc.), egg products, snacks (candy, chips, gum, chocolate, etc.), meats, fruits, and vegetables.

A composition comprising an encapsulated cannabinoid compound can be administered transdermally, such as via a patch. The compositions of the present disclosure can be administered intravenously. The compositions of the present disclosure can be administered topically. The compositions of the present disclosure can be administered via topical exposure to an aqueous solution, such as immersing a subject in a float tank. The compositions of the present disclosure can be formulated as a bath salt or liquid bath product, which can be dissolved or dispersed in water (e.g., a bath) for skin exposure, for example by immersion of the subject. Compositions comprising an encapsulated cannabinoid compound for topical or transdermal application can be provided as cosmetics or personal care products, such as soaps (e.g., solid, bar, liquid, or foaming), hand sanitizer, lotions, massage oils masks, makeup, moisturizers, sunscreen, toothpaste, mouth wash, or throat spray.

To reduce oxidative stress in a cell, a composition comprising an encapsulated cannabinoid compound can be administered using a variety of different mechanisms. The composition may be nebulized, delivered in an aerosolized an aerosol spray preparation from a pressurized pack, or from a dry powder inhaler. Suitable propellants that can be used in a nebulizer include, for example, dichlorodifluoro-methane, trichlorofluoromethane, dichlorotetrafluoroethane and carbon dioxide. The dosage can be determined by providing a valve to deliver a regulated amount of the compound in the case of a pressurized aerosol.

The composition can be administered in a variety of forms to a subject to reduce cellular oxidative stress. The composition can be administered as a dry powder. For example, an oil-based composition (e.g., hemp oil) can be combined with a drying or powdering agent, such as cyclodextrin. In some cases, a powder composition can be provided on its own. In other cases, a powder composition can be provided in another product or composition, such as a food product or composition, cosmetic product, or other products and compositions such as those disclosed herein.

A composition comprising an encapsulated cannabinoid compound can be administered in any suitable form, including but not limited to a liquid form, a gel form, a semiliquid (e.g., a liquid, such as a viscous liquid, containing some solid) form, a semi-solid (a solid containing some liquid) form, or a solid form. Compositions can be provided in, for example, a tablet form, a capsule form, a food form a chewable form, a non-chewable form, a transbuccal form, a sublingual form, a slow-release form, a non-slow-release form, a sustained release form, or a non-sustained-release form.

Compositions comprising an encapsulated cannabinoid compound for inhalation or insufflation include solutions and suspensions in pharmaceutically acceptable, aqueous or organic solvents, or mixtures thereof, and powders. The liquid or solid compositions may contain suitable excipients as set out above. Preferably the compositions of the present invention are administered by the oral, intranasal or respiratory route for local or systemic effect. Compositions in acceptable solvents may be nebulized by use of inert gases. Nebulized solutions may be inhaled directly from a nebulizing device or a nebulizing device may be attached to a face mask, tent or intermittent positive pressure breathing machine. Solution, suspension or powder compositions may be administered orally or nasally from devices that deliver the formulation in an appropriate manner.

The compositions of the present disclosure can comprise an additional agent or agents, whether active or passive. Examples of such an agent include a sweetening agent, a flavoring agent, a coloring agent, a filling agent, a binding agent, a lubricating agent, an excipient, a preservative, or a manufacturing agent. Additional pharmaceutically acceptable excipients (in the case of pharmaceuticals) or other additives (for non-pharmaceutical applications) can be added to the composition. For example, if desired, any generally accepted soluble or insoluble inert pharmaceutical filler (diluent) material can be included in the final product (e.g., a solid dosage form). Such inert pharmaceutical filler can comprise a monosaccharide, a disaccharide, a polyhydric alcohol, inorganic phosphates, sulfates or carbonates, and combinations thereof. Examples of suitable inert pharmaceutical fillers include sucrose, dextrose, lactose, xylitol, fructose, sorbitol, calcium phosphate, calcium sulfate, calcium carbonate, microcrystalline cellulose, and combinations thereof. An effective amount of any generally accepted pharmaceutical lubricant, such as calcium or magnesium soaps, can be added.

In a related but separate aspect, the present disclosure provides a food composition comprising an effective amount of an encapsulated cannabinoid for reducing oxidative stress of a cell and a food carrier. Any type, amount, or form of composition comprising an encapsulated cannabinoid disclosed herein is applicable for preparing the food composition disclosed herein.

A food composition or food product can comprise a food bar, including but not limited to granola bars, protein bars, candy bars, and energy bars. A food composition or food product can comprise a cereal product, including but not limited to oatmeal, flour (e.g., wheat flour, rice flour, corn flour, barley flour), breakfast cereal, granola, bread, pasta, rice cakes, and popcorn. A food composition or food product can comprise a bakery product, including but not limited to bread, pastries, brownies, cakes, pies, donuts, crackers, and muffins. A food composition or food product can comprise a dairy product, including but not limited to milk, fermented milk, curd, whey, yogurt, cream, cheese, butter, clarified butter, ghee, and ice cream. A food composition or food product can comprise a nut butter or seed butter, including but not limited to peanut butter, almond butter, cashew butter, hazelnut butter, macadamia nut butter, pecan butter, pistachio butter, walnut butter, pumpkin seed butter, sesame seed butter, soybean butter, and sunflower seed butter. A food composition or food product can comprise an oil (e.g., a cooking oil), including but not limited to olive oil, coconut oil, vegetable oil, canola oil, corn oil, peanut oil, sunflower seed oil, almond oil, avocado oil, rice bran oil, cottonseed oil, flaxseed oil, linseed oil, grape seed oil, hemp oil, mustard oil, macadamia oil, palm oil, tea seed oil, walnut oil, margarine, lard, butter, clarified butter, ghee, or tallow. A food composition or food product can comprise sports food products such as energy gels, sports drinks, energy powders, energy bars, energy shots, protein powders, and protein drinks (e.g., protein shakes). A food composition or food product can comprise a beverage, including but not limited to water, electrolyte drinks, soda, coconut water, tea (e.g., Jun tea, black tea, green tea, white tea, herbal tea), coffee, a soft drink, an alcoholic beverage (e.g., cocktail, liquor, spirits, beer, wine, malt beverage), water, juice (e.g., apple juice, orange juice, tomato juice, vegetable juice, cranberry juice), a sports drink, electrolyte-enriched water, vitamin-enhanced water, a hangover-recovery drink, milk (e.g., dairy-based milk, coconut milk, almond milk, soy milk, hemp milk, rice milk, oat milk, cashew milk, hazelnut milk), and yogurt. A food composition or food product can comprise a fungus or fermented food or drink, including but not limited to kifir (kefir), jun, amasi, amazake, appam, ayran, doogh, bagoong, brem, cheonggukjang, chicha, kombucha, fermented bean curd, kimchi, lassi, miso, poi, yakult, and yogurt.

Compositions of the present disclosure can comprise pet or other animal products, such as animal food (e.g., dog food, cat food), treats, and nutritional supplements (e.g., liquids, sprays, or powders for application to food or water). These compositions can be formulated for or administered to domestic or pet animals (e.g., dogs, cats, small mammals, birds), livestock and other farm animals (e.g., cows, pigs, horses, sheep, goats), zoo animals, or any other vertebrates. Compositions for administration to animals can be formulated with encapsulated cannabinoid-rich oil or non-encapsulated cannabinoid-rich oil, alone or in combination with essential oils, terpenes, and other components described herein. Compositions for administration to animals can be mixed into feed or water, prepared for spraying application (e.g., mixed in glycerin), for intravenous administration (e.g., in a syringe or an IV bag), in salves, vitamins, liquid vitamin pumps, treats, or other forms.

A food composition can be formulated and/or packaged such that a composition comprising an encapsulated cannabinoid compound can be administered to reduce cellular oxidative in a variety of extreme environmental conditions. For instance, a food composition may be formulated with the appropriate food carrier and packaging such that it may be consumed by an astronaut engaged in space travel, where there may be high levels of oxidative stress from solar radiation, low wavelength electromagnetic radiation, and/or cosmic rays. Packaged food for consumption by astronauts may also be subject to ionizing radiation for sterilization purposes. Ionizing radiation is another source of cellular oxidative stress.

Alternatively or in addition, a food composition may be formulated with the appropriate food carrier and packaging such that it may be consumed during any long distance travel involving any environment, such as underground, underwater, high altitude Earth atmosphere, low altitude Earth atmosphere, non-Earth atmospheres, outer space atmosphere, and/or other environments (or atmospheres).

To accommodate such conditions, food composition can be packaged for consumption in low gravity conditions. Low gravity conditions may refer to conditions where a subject experiences reduced gravitational pull from the Earth that the subject may experience at sea level. Low gravity conditions may refer to microgravity conditions. Under microgravity conditions, the magnitude of gravitational force experienced by a subject is less than at sea level. Under such low gravity conditions, a subject may appear to be weightless. Low gravity conditions may refer to conditions where the force of gravity on a subject is about 90% or less than at sea level. The magnitude of a gravitational force ($F_{grav}$) between two objects may be defined by the following expression:

$$F_{grav} = G\frac{M_1 m_2}{r^2}$$

where $M_1$ and $m_2$ are masses of two bodies, G is the gravitational constant (6.67E-11 m$^3$/s$^2$kg), and r is the distance separating the bodies' centers. Low gravity conditions may refer to conditions where a gravitational force on a subject having a mass is reduced relative to sea level on Earth by at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or more. For instance, for a subject engaged in space travel, the variables $M_1$ and $m_2$ may be the masses of Earth (5.97 x 10$^{24}$ kg) and the subject respectively, r may be distance from the center of the earth to the center of the subject, and G is a constant.

For space travel, packaging of a food or food composition comprising the encapsulated cannabinoid compound can be configured to enable an astronaut to effectively and/or efficiently consume food under low gravity conditions. The packaging may be configured such that the food will not readily disperse into space when consumed, thereby enabling an astronaut engaged in space travel to effectively reduce his or her oxidative stress.

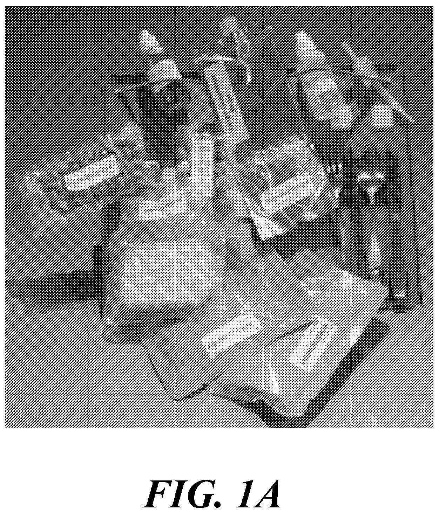

Packaging of a food composition comprising the encapsulated cannabinoid may take the form of several conformations. Packaging may be configured for single service. Packaging may be disposable. As non-limiting examples, packaging may be a pouch, a can, a flexible container, or a sealed tube. Food packaging may be made of any of a variety of materials including Mylar®, Aclar®, polyethylene, foil, and aluminum. Food compositions may be packaged in a retort pouch or a flexible bowl with a lid, food condiment pouches, or full panel pull out cans. Food composition packaging may incorporate utensils such as forks, spoons, knives, or scissors. Packaging may be configured for partial removal to facilitate consumption. For example, a package may remain sealed during rehydration or reheating; for consumption, a portion of the package may be removed. Packaging may be configured for the rehydration of dehydrated food composition powders. For instance, food and beverage packaging may additionally comprise a septum adapter to enable dehydrated food to be rehydrated. Sealed containers comprising food compositions may be configured to be secured in a tray. **FIG. 1A** provides an example of a variety of packaged space foods configured to be secured on a tray. Dry, powdered, or foods subject to crumbling may be coated in gelatin to reduce crumbling. Oxygen may be removed from a package and replaced with a nitrogen gas and an oxygen scavenger. Packages may be flushed with nitrogen gas before sealing. Packages may be sealed with a vacuum seal. The package may be sealed at about 15 to about 35 inches of Hg vacuum.

Packaging for a beverage food composition may also be configured for a low gravity or microgravity environment.

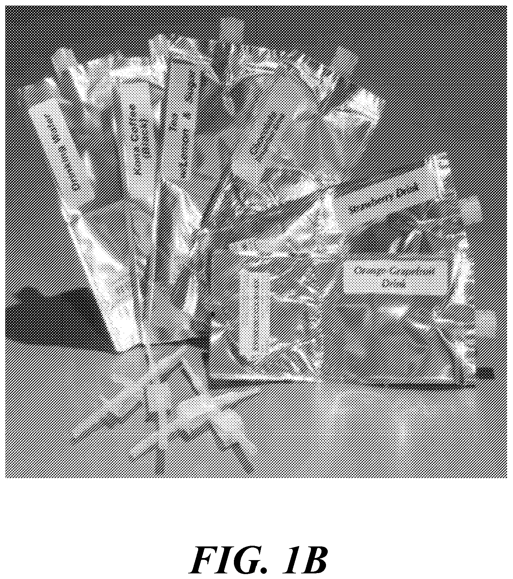

A beverage package may comprise a straw having clamps that prevent liquids from escaping the beverage package. Beverage packaging may comprise a foil laminate. **FIG. 1B** provides an example of foil laminate beverage packaging. A beverage package may comprise a dehydrated powder that may be rehydrated. Beverage packaging may be flushed multiple times with nitrogen. Beverages may be consumed from a clamped straw.

Alternatively or in addition, packaging for a beverage food composition may also be configured for a low pressure environment. Alternatively or in addition, packaging for a beverage food composition may also be configured for a high pressure environment.

A food composition comprising an effective amount of an encapsulated cannabinoid compound may be included in a kit. A kit may be configured for consumption for a subject under a variety of conditions. The conditions may be extreme, such as for space travel, where a subject may be subjected to high oxidative stress. Other examples of extreme conditions may include high altitude environments, extreme temperatures, ultraviolet ray (UV) exposure or environments where a subject many be exposed to elevated levels of radiation or carcinogens.

In addition to reducing oxidative stress, administering the compositions of the present disclosure to a subject can provide one or more additional beneficial effects. Beneficial effects can include but are not limited to pain relief, reduced bacterial growth, reduced blood sugar levels, improved blood lipid and cholesterol profiles, increased fat burning, reduced appetite, stimulated appetite, reduced vomiting or nausea, reduced seizures or convulsions, antifungal effects, reduced inflammation, reduced arthritis (e.g., rheumatoid arthritis), reduced insomnia or aided sleep, reduced arterial blockage, inhibited cancer cell growth, improved psoriasis, tranquilizing effects, antispasmodic effects, reduced anxiety, bone growth promotion, reduced intestinal contractions, and nervous system protection.

The compositions described herein can provide several advantages, including but not limited to increased shelf stability, increased bioavailability, increased bioactivity, and delayed release. The compositions described herein, when administered to a subject, can have various release profiles, half-lives, and metabolic characteristics. The subject compositions can comprise a plurality of capsules, wherein an individual capsule in the plurality is characterized by exhibiting at least one of: (a) a sigmoidal release profile of the at least one cannabinoid compound; (b) a plasma half-life of the at least one cannabinoid compound greater than twice that of the at least one cannabinoid compound in non-encapsulated form; (c) a first pass metabolism of the at least one cannabinoid compound reduced by at least 50% compared to the at least one cannabinoid compound in non-encapsulated form; d) a rate of excretion of the at least one cannabinoid compound from a subject's body reduced by at least 20% compared to the at least one cannabinoid compound in non-encapsulated form; or (e) a degradation rate at an ambient temperature of at least 20 °C of the at least one cannabinoid compound of less than about 50% of a degradation rate of the at least one cannabinoid compound in non-encapsulated form.

The compositions described herein can have a shelf half-life of at least about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200, 210, 240, 270, 300, 330, or 360 days. In some cases, the compositions described herein can have a shelf half-life of at least about 1, 2, 3, 4, or 5 years. Compositions in encapsulated form can be characterized by a cannabinoid degradation rate at an ambient temperature of at least 20 °C of at least 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% less than the degradation rate of a non-encapsulated cannabinoid composition.

Cannabinoid compositions in encapsulated form can be characterized by a plasma half-life in a subject of at least 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, or 5.0 times that of a non-encapsulated cannabinoid composition. Plasma half-life of a composition can be determined experimentally by administering the composition to a subject, taking plasma samples from a subject at multiple time points, and measuring the concentration of the compound or compounds of interest in those plasma samples. The concentration of the compound or compounds of interest will reach a peak value in the plasma, then fall as the compound or compounds are metabolized, degraded, or cleared from the blood stream. The plasma half-life is the time for the plasma concentration value to be halved.

The cannabinoid release profile can be sigmoidal (e.g., having an 'S' shape curve, such as a logistic function). The cannabinoid release profile can be non-sigmoidal. The cannabinoid release profile can be linear. The cannabinoid release profile can be non-linear. The cannabinoid release profile can be instant release. The cannabinoid release profile can be non-instant release. The cannabinoid release profile can be delayed release. The cannabinoid release profile can be constant or sustained release. The cannabinoid release profile can be non-constant or non-sustained release.

Tablets can be formulated in sustained release format. Methods of making sustained release tablets are known in the art; see, for example, U.S. Patent Publication No. 2006/0051416 and U.S. Patent Publication No. 2007/0065512. Gradual-release tablets are known in the art; examples of such tablets are set forth in U.S. Patent No. 3,456,049, for example. A slow- or sustained-release form may delay disintegration or absorption of the composition or one or more components thereof.

In some cases, no more than about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 1 hour of administration to a subject. In some cases, no more than about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 2 hours of administration to a subject. In some cases, no more than about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 3 hours of administration to a subject. In some cases, no more than about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 4 hours of administration to a subject. In some cases, no more than about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 5 hours of administration to a subject. In some cases, no more than about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 6 hours of administration to a subject. In some cases, no more than about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 7 hours of administration to a subject. In some cases, no more than about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% of a cannabinoid compound is released from a capsule within 8 hours of administration to a subject.

A release profile is the relationship between time and the amount of a compound released into a subject or the concentration of the compound within the subject (e.g., within the plasma). Release profiles can be measured in a similar manner to plasma half-life. A composition can be administered to a subject, and samples (e.g., plasma samples or blood samples) can be taken from the subject at multiple time points. The concentration of the compound or compounds of interest can be measured in those samples, and a release profile can be plotted.

Compounds taken up into a subject via the gastrointestinal system can be transported to the liver before entering general circulation. Compounds susceptible to metabolic degradation in the liver can have their activities substantially reduced by the first-pass metabolism through the liver. Encapsulation (e.g., microencapsulation) of compounds can reduce first-pass metabolism of the compounds in the liver. Compositions in encapsulated form can be characterized by a first pass cannabinoid metabolism in a subject of at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% less than that of a non-encapsulated cannabinoid composition. Compositions in encapsulated form can be characterized by a cannabinoid excretion rate from a subject of at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, or 99% less than that of a non-encapsulated cannabinoid composition.

The compositions described herein, when administered to a subject, can have improved bioavailability, bioactivity, or both. Bioavailability is the fraction of an administered dosage of unchanged compound that reaches systemic circulation. Cannabinoid compositions in encapsulated form can be characterized by a bioavailability in a subject of at least about 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0, 5.5, 6.0, 6.5, 7.0, 7.5, 8.0, 8.5, 9.0, 9.5, 10.0 or more times that of a non-encapsulated cannabinoid composition. Cannabinoid compositions in encapsulated form can be characterized by a bioavailability in a subject of at least about 5%, 10%, 15%, 20%, 25%, 30%, 35%, 40%, 45%, 50%, 55%, 60%, 65%, 70%, 75%, 80%, 85%, 90%, 95%, 96%, 97%,%, 98%, 99%, or 100%. Bioactivity, or biological activity, is the activity exerted by the active ingredient or ingredients in a composition. Cannabinoid compositions in encapsulated form can be characterized by a bioactivity in a subject of at least about 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.5, 4.0, 4.5, 5.0, 5.5, 6.0, 6.5, 7.0, 7.5, 8.0, 8.5, 9.0, 9.5, 10.0 or more times that of a non-encapsulated cannabinoid composition.

Subjects of the present disclosure can include humans and other animals, such as pets (e.g., dogs, cats, birds, small mammals, snakes) and livestock or farm animals (e.g., cows, pigs, horses, sheep, chickens). Compositions of the present disclosure can be useful for veterinary applications.

**Example 1 - Food Composition for Space Travel**

A food composition comprising an effective amount of an encapsulated cannabinoid compound may be carried with an astronaut engaged in space travel. Astronauts are subject to especially high amounts of cellular oxidative stress due to exposure to high amounts of radiation, e.g., solar radiation, low wavelength electromagnetic radiation, and galactic cosmic rays from the earth or space environment. Radiation from the aforementioned sources can cause an imbalance of reactive oxygen species. This leads to lipid peroxidation and damage to DNA and proteins. While elevated, antioxidant defenses are often inadequate to compensate for the cellular effects of increased radiation exposure.

A healthy endocannabinoid system plays a vital role in combatting oxidative stress in high oxidative stress environments such as space travel. A healthy endocannabinoid system maintains homeostasis and prevents diseases that can result from endocannabinoid deficiency. For example, endocannabinoid deficiency may result in common diseases such as migraines and fibromyalgia.

Administering a food composition comprising an encapsulated cannabinoid compound reduces cellular oxidative stress experienced by the astronaut engaged in space travel. The food composition is consumed daily, weekly, or monthly during, before, and after space flight. The food composition is consumed before space flight to develop a healthy endocannabinoid system. The food composition is consumed during a spaceflight take-off , when oxidative stress is high. The food composition is consumed during a space flight, e.g., cruising.

The food composition has about 25-2500 mg of encapsulated cannabinoid compound with about 0.3% or less of THC and quillaja extract. Dosing depends on when the food composition is consumed. When cruising, a lower dose (e.g., about 25mg) may be consumed. During events such as takeoff, higher dosages of encapsulated cannabinoid compounds are consumed. (e.g., about100-2500 mg).

Blood samples are taken from the astronaut prior to space travel, during space travel and after space travel. To detect levels of oxidative dress before, during, and after space travel, assays to detect oxidative stress in are used. To assess oxidative stress and effects on cell membrane composition, lipid extraction assays are used to assess lipid composition (e.g., fatty acids, cholesterol, and phospholipids) and lipid peroxidation. To assess lipid perioxidation resulting from oxidative stress, fluorimetric or colorimetric measurement of thiobarbituric acid-reactive substances (TBARS) are used to detect malondialdehydes (MDA) produced from the decomposition of unstable peroxides derived from polyunsaturated fatty acids. The activity of antioxidant enzymes are determined using superoxide dismutase, catalase, glutathione reductase, and glutathione peroxidase activity assays. Total glutathioneare also be assayed.

A reduced level of cellular oxidative stress can be indicated by TBARS fluorimetric or colorimetric assays. An increase in antioxidant activity as indicated by total glutathione and enzymatic activity assays is ascertained.

**Example 2 - Preparation of a Microencapsulated Composition**

Formulations comprising quillaja extract (e.g., Q Natural), hemp oil, water, and optionally sodium alginate were prepared using a microfluidic fluid processor (e.g., Microfluidizer from Microfluidics/IDEX Corporation). Formulations were prepared as described in Table 1. Test 1 was prepared with 60 g of quillaja extract (e.g., Q Natural), 80 g of hemp oil, and 100 g of water, at an operating pressure of 30,000 psi in the microfluidic fluid processor. Test 2 was prepared with 10 g of quillaja extract (e.g., Q Natural), 15 g of hemp oil, 198 g of water, and 2 g of sodium alginate, at an operating pressure of 30,000 psi in the microfluidic fluid processor.

After processing in the microfluidic fluid processor, particle size distribution was analyzed using a laser diffraction particle size analyzer (e.g., Horiba LA950). Optical microscope images were also taken.

Three passes each of Test 1 and Test 2 were conducted, and the tenth percentile (D10), fiftieth percentile (D50), and ninetieth percentile (D90) particle sizes are reported in Table 1. Particle sizes were also analyzed for an unprocessed solution (Test 1, Pass 0).

**Table 1.** Formulation and size distribution information for microencapsulated compositions.

| Test | Pass # | Pressure | Q Natural | Hemp Oil | Water | Sodium alginate | D10 ($\mu$m) | D50 ($\mu$m) | D90 ($\mu$m) |
|------|--------|----------|-----------|----------|-------|-----------------|-------------|-------------|-------------|
| 1 | 0 | 30,000 psi | 60 g | 80 g | 100 g | - | 1.9737 | 7.117 | 35.703 |
|   | 1 |            |      |      |       | - | 0.0768 | 0.1296 | 0.2881 |
|   | 2 |            |      |      |       | - | 0.0697 | 0.1096 | 0.1791 |
|   | 3 |            |      |      |       | - | 0.0929 | 0.1405 | 0.2202 |
| 2 | 1 | 30,000 psi | 10 g | 15 g | 198 g | 2 g | 0.1171 | 0.1965 | 2.0719 |
|   | 2 |            |      |      |       |     | 0.0688 | 0.1097 | 0.2209 |
|   | 3 |            |      |      |       |     | 0.099 | 0.1583 | 1.3443 |

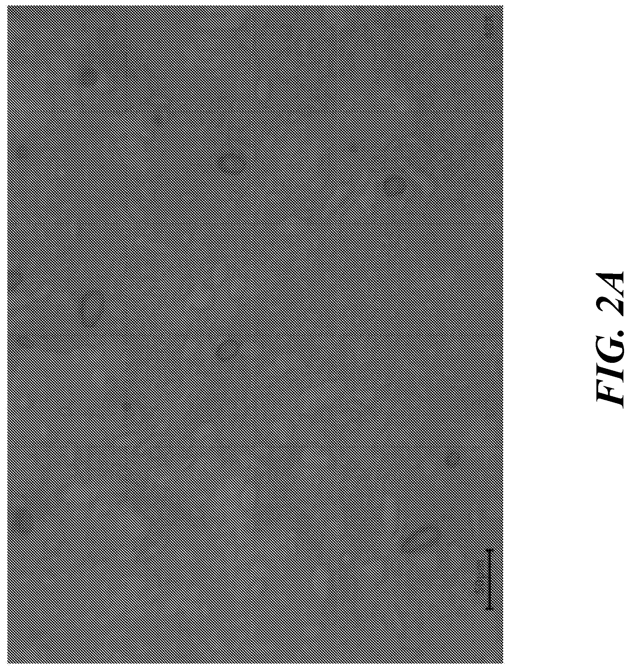

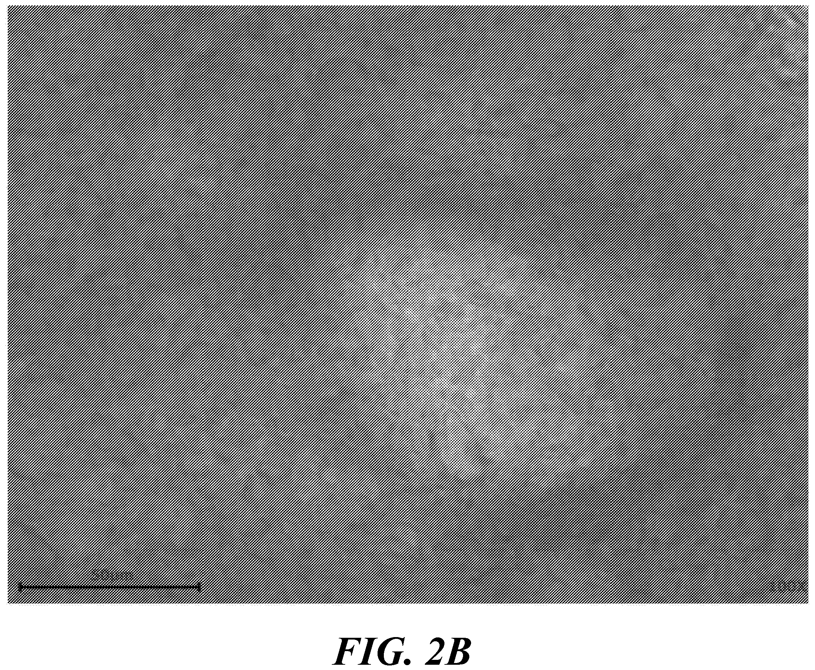

**FIG. 2A** shows a 400x magnification micrograph image of an unprocessed quillaja extract, hemp oil, and water composition (Test 1, Pass 0), with a 50 $\mu$m scale bar. **FIG. 2B** shows a 1000x magnification micrograph image of an unprocessed quillaja extract, hemp oil, and water composition (Test 1, Pass 0), with a 50 $\mu$m scale bar.

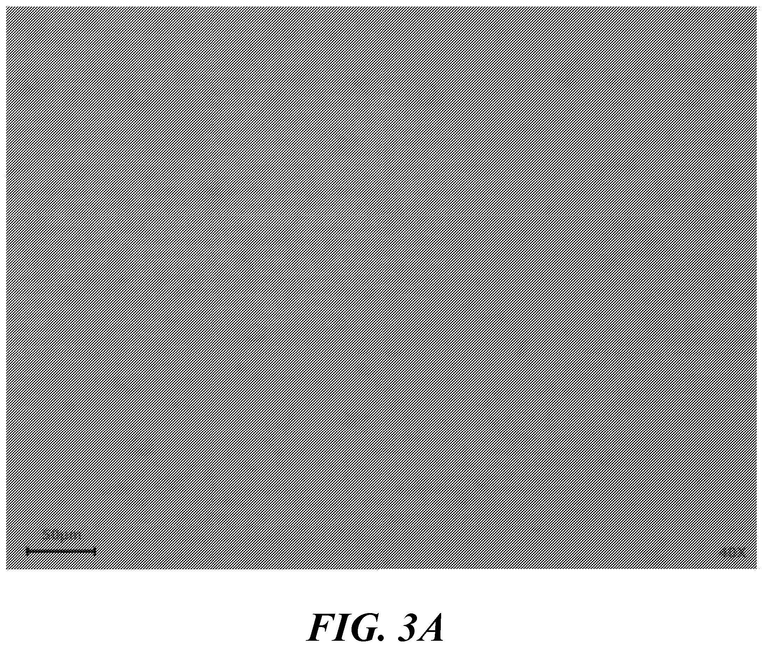

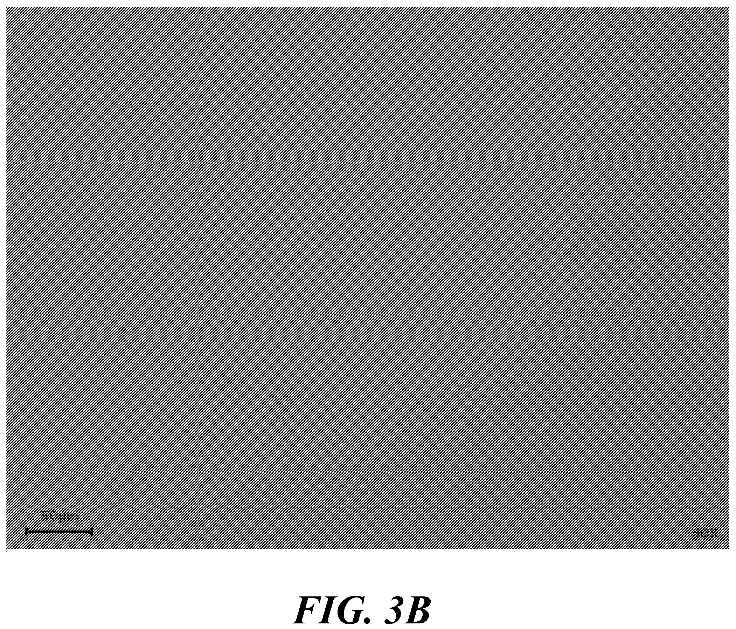

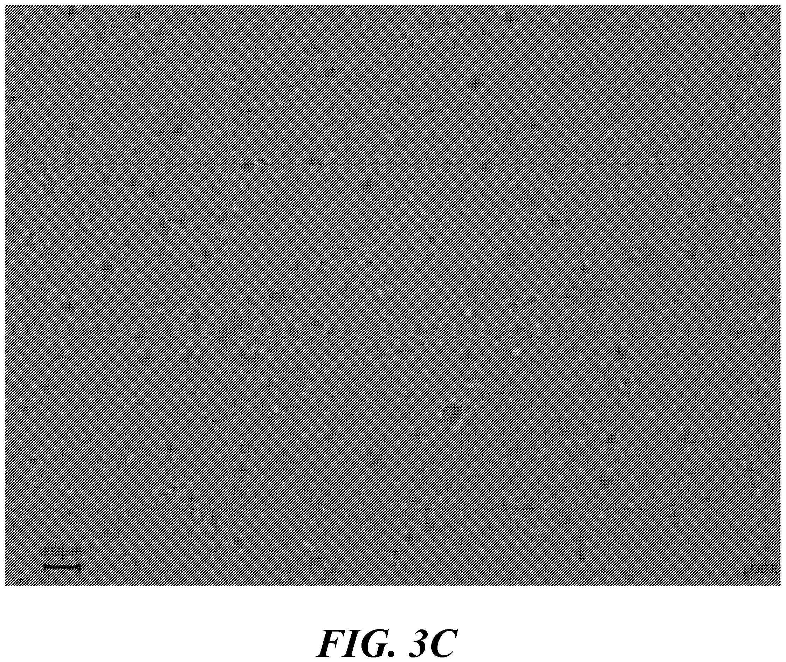

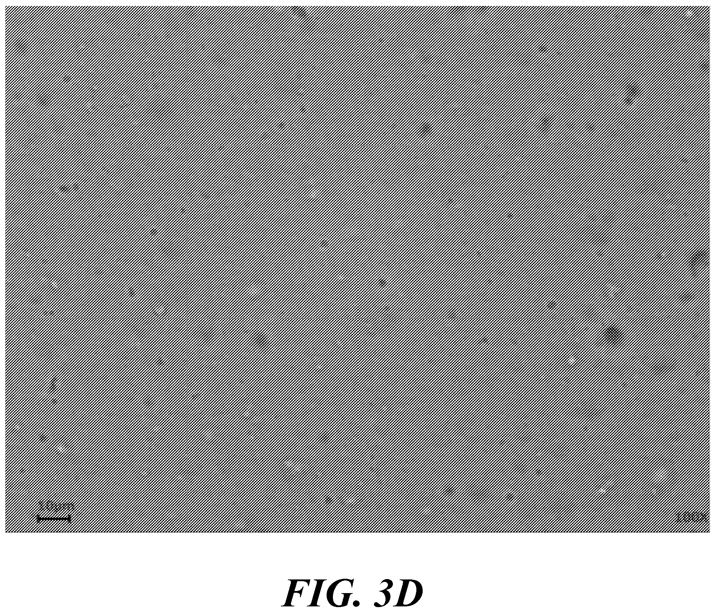

**FIG. 3A** shows a 400x magnification micrograph image of a quillaja extract, hemp oil, and water composition (Test 1, Pass 1), with a 50 $\mu$m scale bar. **FIG. 3B** shows a 400x magnification micrograph image of a quillaja extract, hemp oil, and water composition (Test 1, Pass 2), with a 50 $\mu$m scale bar. **FIG. 3C** shows a 1000x magnification micrograph image of a quillaja extract, hemp oil, and water composition (Test 1, Pass 2), with a 10 $\mu$m scale bar. **FIG. 3D** shows a 1000x magnification micrograph image of a quillaja extract, hemp oil, and water composition (Test 1, Pass 3), with a 10 $\mu$m scale bar.

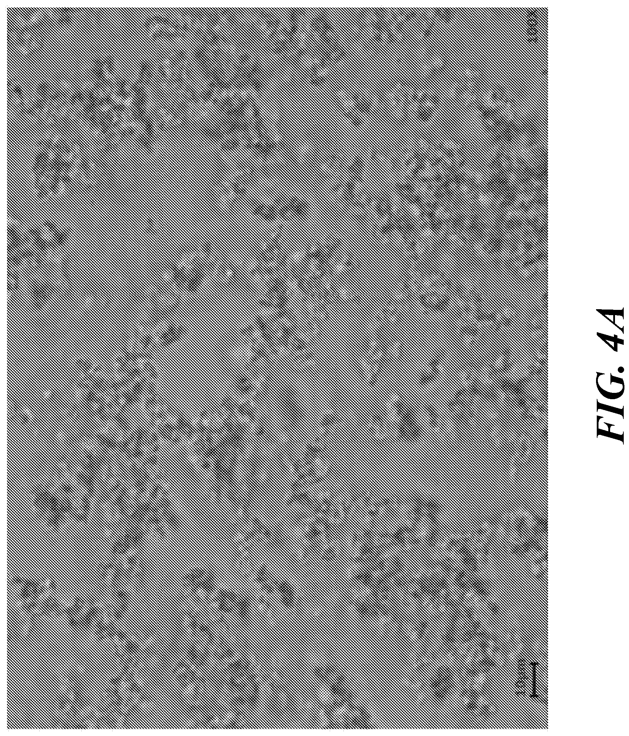

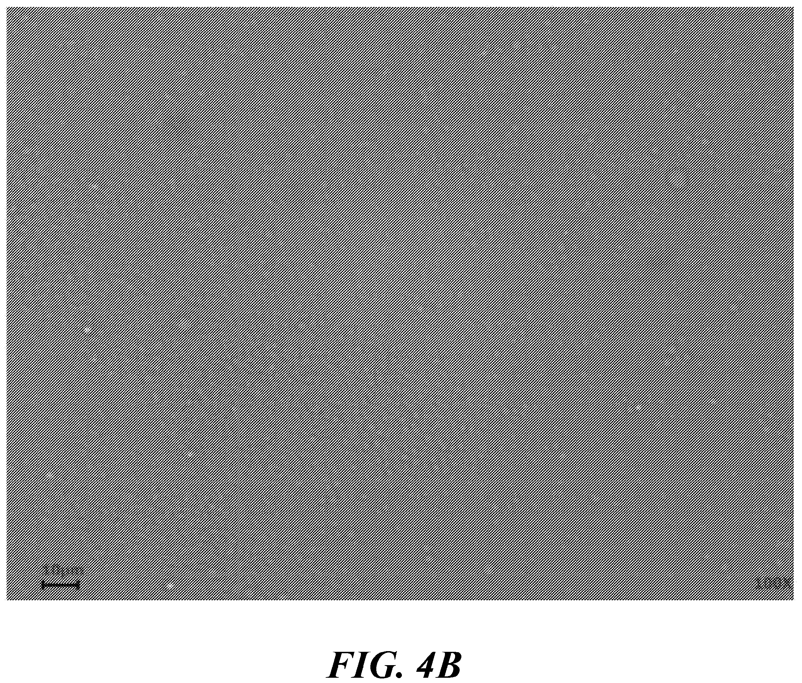

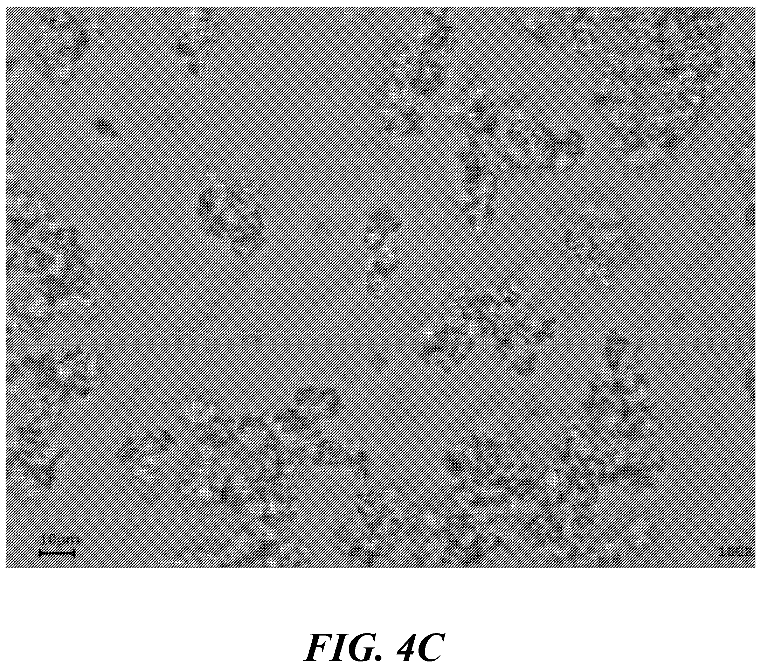

**FIG. 4A** shows a 1000x magnification micrograph image of a quillaja extract, hemp oil, water, and sodium alginate composition (Test 2, Pass 1), with a 10 μm scale bar. **FIG. 4B** shows a 1000x magnification micrograph image of a quillaja extract, hemp oil, water, and sodium alginate composition (Test 2, Pass 2), with a 10 μm scale bar. **FIG. 4C** shows a 1000x magnification micrograph image of a quillaja extract, hemp oil, water, and sodium alginate composition (Test 2, Pass 3), with a 10 μm scale bar.

**Claims**

1. A composition for use in a method for reducing oxidative stress in a subject suffering or suspected of suffering from a health condition, comprising:
an antioxidant and a microencapsulated cannabinoid compound and a terpene, wherein the terpene is microencapsulated with the cannabinoid compound.

2. The composition for use of claim 1, wherein said antioxidant is an enzymatic antioxidant.

3. The composition for use of claim 1, wherein:

   (a) said subject is travelling in outer space; or
   (b) said subject is exposed to one or more type of radiation selected from the group consisting of: solar radiation, low wavelength electromagnetic radiation, galactic cosmic rays, and a combination thereof.

4. The composition for use of claim 1, wherein

   (a) said composition is administered orally;
   (b) said composition is a food or beverage for oral administration;
   (c) said composition is administered by inhalation;
   (d) said composition is nebulized for inhalation; or
   (e) said composition is administered transdermally.

5. The composition for use of claim 1, wherein:

   (a) said composition is administered topically; or
   (b) said composition is water soluble.

6. The composition for use of claim 1, wherein an amount of said composition is for use in reducing oxidative stress in said subject, wherein a level of oxidative stress of said subject has been determined prior to said use.

7. The composition for use of claim 6, wherein said amount is determined based on said level.

8. The composition for use of claim 1, wherein an additional or reduced amount of said composition is for use in reducing oxidative stress in said subject, wherein a level of oxidative stress of said subject has been determined prior to said use, and wherein said subject has received an initial amount of said composition prior to said use.

9. The composition for use of claim 8, wherein said level of oxidative stress has been determined based on a measurement of one or more reactive oxygen species or one or more antioxidants in said subject.

10. A food composition for use in reducing oxidative stress in a cell of a subject, comprising: (a) an antioxidant and a microencapsulated cannabinoid compound and a terpene, wherein the terpene is microencapsulated with the cannabinoid compound; and (b) a food carrier.

11. The food composition for use of claim 10, wherein the food composition:

   (a) is packaged as a beverage;
   (b) is packaged as a solid food;
   (c) is packaged as a semi-solid food; or
   (d) is packaged for microgravity conditions.

12. The food composition for use of claim 10, wherein the food composition is packaged for microgravity conditions.

**13.** The food composition for use of claim 10, wherein the terpene is quillaja.

**14.** The composition for use of claim 1, wherein said terpene is quillaja.

**Patentansprüche**

**1.** Zusammensetzung zur Verwendung in einem Verfahren zum Reduzieren von oxidativem Stress in einem Individuum, das an einer Erkrankung leidet oder bei dem die Vermutung dafür besteht, umfassend:
ein Antioxidans und eine mikroverkapselte Cannabinoidverbindung und ein Terpen, wobei das Terpen mit der Cannabinoidverbindung mikroverkapselt ist.

**2.** Zusammensetzung zur Verwendung nach Anspruch 1, wobei das Antioxidans ein enzymatisches Antioxidans ist.

**3.** Zusammensetzung zur Verwendung nach Anspruch 1, wobei:

(a) sich das Individuum im Weltall bewegt; oder
(b) das Individuum einer oder mehreren Arten von Strahlung ausgesetzt ist, die aus der aus Folgenden bestehenden Gruppe ausgewählt ist: Sonnenstrahlung, elektromagnetischer Strahlung mit niedriger Wellenlänge, galaktischer kosmischer Strahlung und einer Kombination daraus.

**4.** Zusammensetzung zur Verwendung nach Anspruch 1, wobei:

(a) die Zusammensetzung oral verabreicht wird;
(b) die Zusammensetzung ein Nahrungsmittel oder ein Getränk zur oralen Verabreichung ist;
(c) die Zusammensetzung durch Inhalation verabreicht wird;
(d) die Zusammensetzung zur Inhalation vernebelt wird; oder
(e) die Zusammensetzung transdermal verabreicht wird.

**5.** Zusammensetzung zur Verwendung nach Anspruch 1, wobei:

(a) die Zusammensetzung topisch verabreicht wird; oder
(b) die Zusammensetzung wasserlöslich ist.

**6.** Zusammensetzung zur Verwendung nach Anspruch 1, wobei eine Menge der Zusammensetzung zur Verwendung bei der Verringerung von oxidativem Stress in dem Individuum dient, wobei ein Ausmaß von oxidativem Stress in dem Individuum vor der Verwendung bestimmt wurde.

**7.** Zusammensetzung zur Verwendung nach Anspruch 6, wobei die Menge basierend auf dem Ausmaß bestimmt wird.

**8.** Zusammensetzung zur Verwendung nach Anspruch 1, wobei eine zusätzliche oder reduzierte Menge der Zusammensetzung zur Verwendung beim Verringern von oxidativem Stress in dem Individuum dient, wobei ein Ausmaß von oxidativem Stress des Individuums vor der Verwendung bestimmt wurde, und wobei das Individuum eine anfängliche Menge der Zusammensetzung vor der Verwendung erhalten hat.

**9.** Zusammensetzung zur Verwendung nach Anspruch 8, wobei das Ausmaß von oxidativem Stress basierend auf einer Messung einer oder mehrerer reaktiver Sauerstoffspezies oder eines oder mehrerer Antioxidantien in dem Individuum bestimmt wurde.

**10.** Nahrungsmittelzusammensetzung zur Verwendung bei der Verringerung von oxidativem Stress in einer Zelle eines Individuums, umfassend: (a) ein Antioxidans und eine mikroverkapselte Cannabinoidverbindung und ein Terpen, wobei das Terpen mit der Cannabinoidverbindung mikroverkapselt ist; und (b) einen Nahrungsmittelträger.

**11.** Nahrungsmittelzusammensetzung zur Verwendung nach Anspruch 10, wobei die Nahrungsmittelzusammensetzung:

(a) als Getränk verpackt ist;
(b) als festes Nahrungsmittel verpackt ist;

(c) als halbfestes Nahrungsmittel verpackt ist; oder
(d) für Mikroschwerkraftsbedingungen verpackt ist.

**12.** Nahrungsmittelzusammensetzung zur Verwendung nach Anspruch 10, wobei die Nahrungsmittelzusammensetzung für Mikroschwerkraftsbedingungen verpackt ist.

**13.** Nahrungsmittelzusammensetzung zur Verwendung nach Anspruch 10, wobei das Terpen Quillaja ist.

**14.** Zusammensetzung zur Verwendung nach Anspruch 1, wobei das Terpen Quillaja ist.

**Revendications**

**1.** Composition pour utilisation dans un procédé de réduction du stress oxydatif chez un sujet souffrant ou soupçonné de souffrir d'un état de santé, comprenant :
un antioxydant et un composé cannabinoïde microencapsulé et un terpène, dans laquelle le terpène est microencapsulé avec le composé cannabinoïde.

**2.** Composition pour utilisation selon la revendication 1, dans laquelle ledit antioxydant est un antioxydant enzymatique.

**3.** Composition pour utilisation selon la revendication 1, dans laquelle :

(a) ledit sujet voyage dans l'espace ; ou
(b) ledit sujet est exposé à un ou plusieurs types de rayonnement choisis dans le groupe constitué de : rayonnement solaire, rayonnement électromagnétique de faible longueur d'onde, rayons cosmiques galactiques, et une combinaison de ceux-ci.

**4.** Composition pour utilisation selon la revendication 1, dans laquelle

(a) ladite composition est administrée par voie orale ;
(b) ladite composition est un aliment ou une boisson pour administration orale ;
(c) ladite composition est administrée par inhalation ;
(d) ladite composition est nébulisée pour inhalation ; ou
(e) ladite composition est administrée par voie transdermique.

**5.** Composition pour utilisation selon la revendication 1, dans laquelle :

(a) ladite composition est administrée par voie topique ; ou
(b) ladite composition est soluble dans l'eau.

**6.** Composition pour utilisation selon la revendication 1, dans laquelle une quantité de ladite composition est destinée à être utilisée pour réduire le stress oxydatif chez ledit sujet, dans laquelle un niveau de stress oxydatif dudit sujet a été déterminé avant ladite utilisation.

**7.** Composition pour utilisation selon la revendication 6, dans laquelle la quantité est déterminée sur la base dudit niveau.

**8.** Composition pour utilisation selon la revendication 1, dans laquelle une quantité supplémentaire ou réduite de ladite composition est destinée à être utilisée pour réduire le stress oxydatif chez ledit sujet, dans laquelle un niveau de stress oxydatif dudit sujet a été déterminé avant ladite utilisation, et dans laquelle ledit sujet a reçu une quantité initiale de ladite composition avant ladite utilisation.

**9.** Composition pour utilisation selon la revendication 8, dans laquelle ledit niveau de stress oxydatif a été déterminé sur la base d'une mesure d'une ou plusieurs espèces d'oxygène réactives ou d'un ou plusieurs antioxydants chez ledit sujet.

**10.** Composition alimentaire pour utilisation pour réduire le stress oxydatif dans une cellule d'un sujet, comprenant : (a) un antioxydant et un composé cannabinoïde microencapsulé et un terpène, dans laquelle le terpène est microencapsulé avec le composé cannabinoïde ; et (b) un support alimentaire.

**11.** Composition pour utilisation selon la revendication 10, dans laquelle la composition alimentaire :

(a) est conditionnée sous la forme d'une boisson ;
(b) est conditionnée sous la forme d'un aliment solide ;
(c) est conditionnée sous la forme d'un aliment semi-solide ; ou
(d) est conditionné pour des conditions de microgravité.

**12.** Composition alimentaire pour utilisation selon la revendication 10, dans laquelle la composition alimentaire est conditionnée pour des conditions de microgravité.

**13.** Composition alimentaire pour utilisation selon la revendication 10, dans laquelle le terpène est du quillaja.

**14.** Composition pour utilisation selon la revendication 1, dans laquelle ledit terpène est du quillaja.

*FIG. 1A*

*FIG. 1B*

*FIG. 2A*

*FIG. 2B*

*FIG. 3A*

*FIG. 3B*

*FIG. 3C*

*FIG. 3D*

*FIG. 4A*

*FIG. 4B*

*FIG. 4C*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62350702
- US 7482152 B
- WO 2016094810 A
- US 20060051416 A
- US 20070065512 A
- US 3456049 A

**Non-patent literature cited in the description**

- **COMELLI et al.** *Phytotherapy research*, 2009, vol. 23, 1678-1684